# EUROPEAN PATENT SPECIFICATION

(11) **EP 2 276 725 B1**
(45) Date of publication and mention of the grant of the patent: **04.07.2012**
(21) Application number: 09730174.1
(22) Date of filing: 30.03.2009
(51) Int. Cl.: C07C 235/06, C07C 235/16, C07D 295/185, C07D 487/04, C07F 15/00, A61K 38/05, A61P 31/12

(54) **NEW RUTHENIUM COMPLEXES AS CATALYSTS FOR METATHESIS REACTIONS**
NEUE RUTHEN-KOMPLEXE ALS KATALYSATOREN FÜR METATHESEREAKTIONEN
NOUVEAUX COMPLEXES DU RUTHÉNIUM COMME CATALYSEURS POUR DES RÉACTIONS DE MÉTATHÈSE

(30) Priority: 11.04.2008 EP 08154367
(43) Date of publication of application: 26.01.2011
(73) Proprietor: F. Hoffmann-La Roche AG, 4070 Basel (CH)
(72) Inventor: PUENTENER, Kurt, CH-4052 Basel (CH); SCALONE, Michelangelo, CH-4127 Birsfelden (CH)
(74) Representative: Rauber, Beat
(86) International application number: PCT/EP2009/053711
(87) International publication number: WO 2009/124853

(56) References cited:
- EP-A- 0 921 129
- WO-A-2004/089974
- WO-A-2005/016944
- WO-A-2005/037214
- WO-A-2008/034522
- US-A- 5 936 100
- US-A1- 2005 267 018
- US-A1- 2006 063 915
- W.T. BRADY ET. AL.: "Intramolecular [2+2] cycloadditions of ketene iminium salts to carbon-carbon double bonds." JOURNAL OF ORGANIC CHEMISTRY, vol. 52, no. 11, 1987, pages 2216-20, XP002526631
- M. BIENICK ET. AL.: "In an Attempt to Provide a Users Guide to the Galaxy of Benzylidene, Alkoxybenzylidene, and indenylidene Ruthenium Olefin Metathesis Catalysts." CHEMISTRY-A EUROPEAN JOURNAL, vol. 14, no. 3, 18 January 2008 (2008-01-18), pages 806-18, XP002526632

## Description

The invention relates to novel metathesis catalysts of the formula 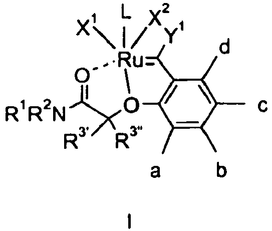 a process for making the same and their use in metathesis reactions such as ring closing (RCM) or cross metathesis.

The invention further relates to a process for the manufacture of a macrocyclic compound of formula 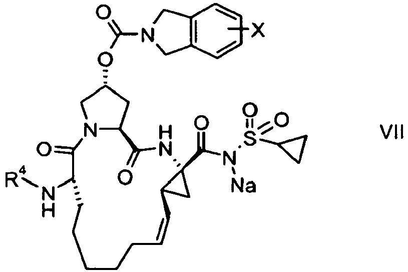 wherein R⁴ is an amino protecting group and X is a halogen atom. Particularly the HCV protease inhibitor compound of the formula 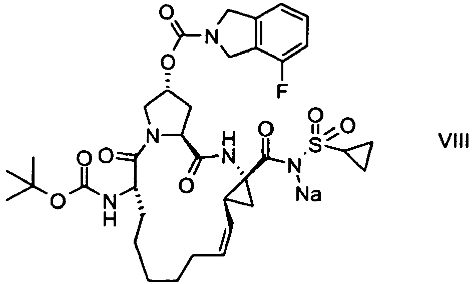 has been nominated for preclinical development.

Metathesis reactions using ruthenium or other transition metal complexes as catalysts are meanwhile well known and have been widely applied in the synthesis of macrocyclic compounds.

For instance the PCT Publication WO 2005/016944 discloses ester type ruthenium metal complexes of formula 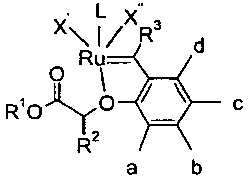 and their application in metathesis reactions.

Also Grela et al. et al in Chem. Eur. J., 2008, 14, 806-818 shows the application of indenylidene ruthenium catalysts in olefin metathesis.

For example the PCT Publication WO 2005/037214 or PCT Publication WO 2007/015824 discloses a RCM of a diene of the formula 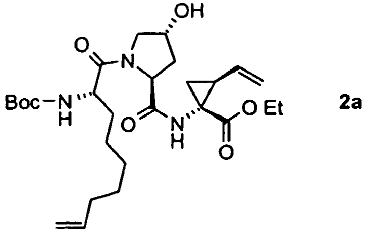 in the presence of a Nolan or Hoveyda catalyst to form the macrocycle of formula

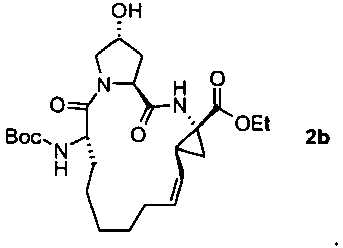

It was found that the RCM as disclosed in the art suffer from a low performance of the reaction due to modest yields and low catalyst selectivity, which translate into low efficiency and high costs.

Object of the present invention therefore was to find superior metathesis catalysts and an improved process which is applicable on technical scale and is able to overcome the disadvantages known in the art.

It was found that the Ru-complexes of formula I have the potential to be useful catalysts in metathesis reactions such as in ring closing metathesis and in cross metathesis reactions.

The compounds of the present invention are characterized by the formula 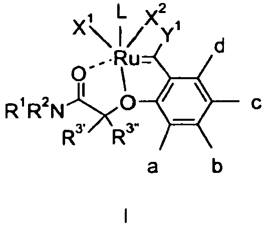 wherein the dotted line either signifies the existence of a bond or no bond;
L is a neutral ligand selected from 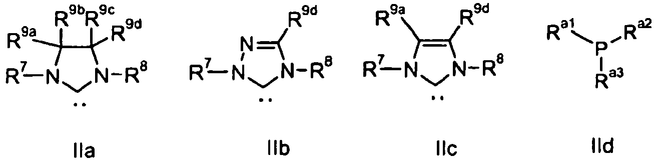 wherein R⁷ and R⁸ independently of each other are C₁₋₆-alkyl, aryl, C₂₋₆- alkenyl or 1-adamantyl and
R^{9a-d} are independently of each other hydrogen, C₁₋₆-alkyl, C₂₋₆- alkenyl or aryl, or R^{9b} and R^{9c} or R^{9a} and R^{9d} taken together form a-(CH₂)₄-bridge;
or in formula IIc R^{9a} and R^{9d} are both halogen;
R^{a1}, R^{a2} and R^{a3} independently of each other are C₁₋₆-alkyl, C₃₋₈-cycloalkyl, aryl, heteroaryl or R^{a1} and R^{a2} or R^{a2} and R^{a3} or R^{a1} and R^{a3} taken together form a 1,5-bridged cyclooctyl group;
X¹ and X² independently of each other are anionic ligands;
Y¹ is hydrogen, C₁₋₆-alkyl, C₃₋₈-cycloalkyl, C₂₋₆-alkenyl, C₂₋₆-alkynyl, C₁₋₆-alkoxy, C₂₋₆-alkenyloxy, C₂₋₆-alkynyloxy, aryloxy, C₁₋₆-alkoxycarbonyl, C₁₋₆-alkylthio, aryl, arylthio, C₁₋₆-alkylsulfonyl, C₁₋₆-alkylsulfinyl;
a, b, c and d independently of each other have the meaning of hydrogen, C₁₋₆-alkyl, halogen-C₁₋₆-alkyl, C₂₋₆-alkenyl, C₂₋₆-alkynyl, C₁₋₆-alkoxy, C₂₋₆-alkenyloxy, C₂₋₆-alkynyloxy, C₁₋₆-alkylcarbonyl, aryl, hydroxy, aryloxy, nitro, C₁₋₆-alkoxycarbonyl, amino, mono-C₁₋₆-alkyl-or di-C₁₋₆-alkylamino, halogen, thio, C₁₋₆-alkylthio, arylthio, C₁₋₆-alkylsulfonyl, C₁₋₆-alkylsulfinyl, arylsulfonyl, SO₃H, C₁₋₆-alkylcarbonyl amino, aryl carbonyl amino, C₁₋₆-alkyl sulfonyl amino, aryl sulfonyl amino, halogen-C₁₋₆-alkyl sulfonyl amino, SO₃-C₁₋₆-alkyl or OSi(C₁₋₆-alkyl)₃ and SO₂-NR'R'' wherein R' and R" independently of each other have the meaning of hydrogen, aryl or C₁₋₆-alkyl or R' and R" together with the N atom form a cycle;
R¹ and R² independently of each other are hydrogen, C₁₋₆-alkyl, C₃₋₈-cycloalkyl, aryl, aryl-C₁₋₆-alkyl or
R¹ and R² together with the N atom form a 5 to 8 member cycle which may contain nitrogen, oxygen or sulfur as additional hetero atom;
R^{3'} and R^{3''} independently of each other are hydrogen, C₁₋₆-alkyl, C₃₋₈-cycloalkyl, aryl, aryl-C₁₋₆-alkyl.

The object could further be reached with the process of the present invention as outlined below.

The process for the manufacture of a macrocyclic compound of formula 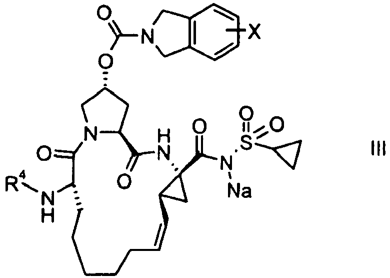 wherein R⁴ is an amino protecting group and X is a halogen atom, comprises the steps
a) subjecting a diene compound of formula 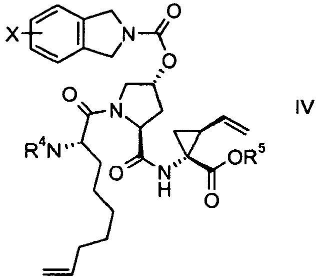 wherein R⁴ is an amino protecting group, R⁵ is C₁₋₄-alkyl and X is halogen to ring closing metathesis reaction in the presence of a compound of the formula I as defined above.
   to form a macrocyclic ester of the formula 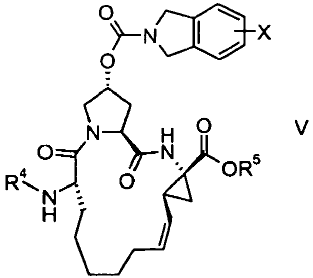 wherein R⁴ is an amino protecting group, R⁵ is C₁₋₄-alkyl and X is halogen;
b) hydrolyzing the macrocyclic ester of formula V in the presence of a base to form the macrocyclic acid of the formula 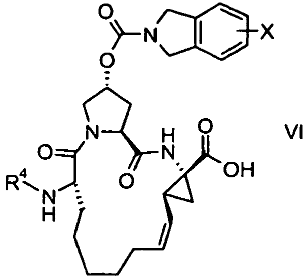 wherein R⁴ is an amino protecting group and X is halogen;
c) forming the macrocyclic sulfonamide of formula 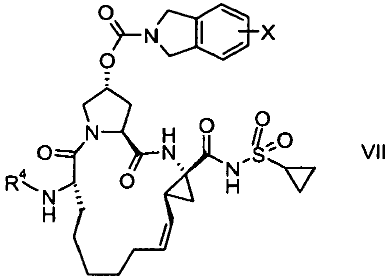 wherein R⁴ is an amino protecting group and X is halogen by coupling the macrocyclic acid of formula VI with cyclopropyl sulfonamide and
d) treating the macrocyclic sulfonamide of formula VII with a sodium base to form the macrocyclic compound of formula III.

The following definitions are set forth to illustrate and define the meaning and scope of the various terms used to describe the invention herein.

The term "amino protecting group" refers to any substituents conventionally used to hinder the reactivity of the amino group. Suitable amino protecting groups are described in Green T., "Protective Groups in Organic Synthesis", Chapter 7, John Wiley and Sons, Inc., 1991, 309-385. Suitable amino protecting groups are Fmoc, Cbz, Moz, Boc, Troc, Teoc or Voc. Preferred amino protecting group, as defined for R⁴ is Boc.

The term "halogen" refers to fluorine, chlorine, bromine and iodine. The preferred halogen for X is fluorine, for X¹ and X² is chlorine.

In a preferred embodiment the moiety of the formula 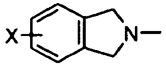 stands for

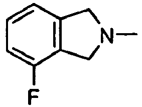

The term "C₁₋₆-alkyl", alone or in combination with other groups, refers to a branched or straight-chain monovalent saturated aliphatic hydrocarbon radical of one to six carbon atoms, preferably one to four carbon atoms. This term is further exemplified by radicals as methyl, ethyl, n-propyl, isopropyl, n-butyl, s-butyl, t-butyl and pentyl or hexyl and its isomers.

The term "C₁₋₄-alkyl" as used in herein for R⁵ refers to a branched or straight-chain monovalent saturated aliphatic hydrocarbon radical of one to four carbon atoms such as methyl, ethyl, n-propyl, isopropyl, n-butyl, s-butyl, t-butyl, preferably to ethyl.

The term "C₂₋₆-alkenyl", alone or in combination with other groups, refers to a branched or straight-chain monovalent unsaturated aliphatic hydrocarbon radical of two to six carbon atoms, preferably two to four carbon atoms. This term is further exemplified by radicals as vinyl, propenyl, butenyl, pentenyl and hexenyl and their isomers. Preferred alkenyl radical is vinyl.

The term "C₂-₆-alkynyl", alone or in combination with other groups, refers to a branched or straight-chain monovalent unsaturated aliphatic hydrocarbon radical of two to six carbon atoms, preferably two to four carbon atoms. This term is further exemplified by radicals as ethynyl, propynyl, butynyl, pentynyl or hexynyl their isomers.

The term "halogen-C₁₋₆-alkyl" refers to a halogen substituted C₁₋₆-alkyl radical wherein halogen has the meaning as above. Preferred "halogen-C₁₋₆-alkyl" radicals are the fluorinated C₁₋₆-alkyl radicals such as CF₃, CH₂CF₃, CH(CF₃)₂, CH(CH₃)(CF₃), C₄F₉.

The term "C₁₋₆-alkoxy" refers to a branched or straight-chain monovalent saturated aliphatic hydrocarbon radical of one to six carbon atoms, preferably 1 to 4 carbon atoms attached to an oxygen atom. Examples of "alkoxy" are methoxy, ethoxy, propoxy, isopropoxy, butoxy, isobutoxy and hexyloxy. Preferred are the alkoxy groups specifically exemplified herein.

The alkyl chain of the alkoxy group can optionally be substituted, particularly mono-, di- or tri-substituted by alkoxy groups as defined above, preferably methoxy, or ethoxy or by aryl groups, preferably phenyl. Preferred substituted alkoxy group is the benzyloxy group.

The term "C₁₋₆-alkyl carbonyl" refers to C₁₋₆-alkyl substituted carbonyl group, preferably to a C₁₋₄-alkycarbonyl group. It includes for example acetyl, propanoyl, butanoyl or pivaloyl. Preferred alkyl carbonyl group is acetyl.

The term "C₁₋₆-alkylthio" refers to the group C₁₋₆-alkyl-S-, preferably C₁₋₄-alkyl e.g. methylthio or ethylthio. Preferred are the alkylthio groups specifically exemplified herein.

The term "arylthio" refers to a group aryl-S-, preferably to phenylthio.

The term "C₁₋₆-alkylsulfonyl" refers to a C₁₋₆-alkyl substituted sulfonyl group, preferably to methylsulfonyl.

The term "C₁₋₆-alkylsulfinyl" refers to a C₁₋₆-alkyl substituted sulfinyl group, preferably to methylsulfinyl.

The term "SO₂- aryl" refers to a sulfonyl substituted aryl radical. Preferred SO₂-aryl radical is SO₂-phenyl.

The term "SO₂-NR'R'' " refers to a sulfonyl group substituted with an amino group NR'R'' wherein R' and R" independently of each other have the meaning of hydrogen or C₁₋₆-alkyl or R' and R" together with the N atom form a cycle, e.g. - (CH₂)₄- or -(CH)₄-. Preferred SO₂-NR'R'' radical is SO₂-N(CH₃)₂.

The term "mono- or di-C₁₋₆-alkyl-amino" refers to an amino group, which is mono- or disubstituted with C₁₋₆-alkyl, preferably C₁₋₄-alkyl. A mono-C₁₋₆-alkyl-amino group includes for example methylamino or ethylamino. The term "di-C₁₋₆-alkyl-amino" includes for example dimethylamino, diethylamino or ethylmethylamino. Preferred are the mono- or di-C₁₋₄-alkylamino groups specifically exemplified herein. It is hereby understood that the term "di-C₁₋₆-alkyl-amino" includes ring systems wherein the two alkyl groups together with the nitrogen atom to which they are attached form a 4 to 7 membered heterocycle which also may carry one further hetero atom selected from nitrogen, oxygen or sulfur.

The term "cycloalkyl" denotes a "C₃₋₈-cycloalkyl" group containing from 3 to 8 carbon atoms, such as cyclopropyl, cyclobutyl, cyclopentyl, cyclohexyl, cycloheptyl or cyclooctyl.

The term "aryl" relates to a phenyl or naphthyl group, which can optionally be mono-, di-, tri- or multiply-substituted by halogen, hydroxy, CN, halogen-C₁₋₆-alkyl, NO₂, NH₂, N(H,alkyl), N(alkyl)₂, carboxy, aminocarbonyl, alkyl, alkoxy, alkylcarbonyl, C₁₋₆-alkylsulfonyl, SO₂-aryl, SO₃H, SO₃-alkyl, SO₂-NR'R'', aryl and/or aryloxy. Preferred aryl group usually is phenyl, however the preference for aryl may differ as indicated hereinafter for certain substituents.

The term "aryloxy" relates to an aryl radical attached to an oxygen atom. The term "aryl" has the meaning as defined above. Preferred aryloxy group is phenyloxy.

The term "arylalkyl" relates to an aryl radical attached to an alkyl group. The term "aryl" has the meaning as defined above. Preferred arylalkyl group is benzyl.

The term "heteroaryl" relates to a heterocyclic aryl radical containing 1 to 3 heteroatoms in the ring with the remainder being carbon atoms. Suitable heteroatoms include, without limitation, oxygen, sulfur, and nitrogen. Exemplary heteroaryl groups include furanyl, thienyl, pyridyl, pyrrolyl, N-alkyl pyrrolo, pyrimidyl, pyrazinyl, imidazolyl, benzofuranyl, quinolinyl, and indolyl. Like the aryl group the heteroaryl group can optionally be mono-, di-, tri- or multiply-substituted by halogen, hydroxy, CN, NO₂, NH₂, N(H,alkyl), N(alkyl)₂, carboxy, aminocarbonyl, alkyl, alkoxy, alkylcarbonyl, C₁₋₆-alkylsulfonyl, SO₂-aryl, SO₃H, SO₃-alkyl, SO₂-NR'R'', aryl and/or aryloxy.

The compounds of formula I 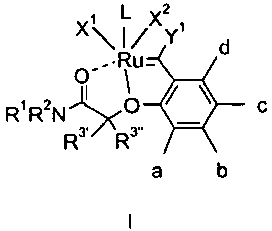 can preferably be characterized by the following definitions.

Where the dotted line signifies the existence of a bond, i.e. the amidic carbonyl group coordinates to ruthenium (II) and generates a hexacoordinated environment.

Compounds of formula I with a hexacoordinated environment are preferred.

Where the dotted line signifies no bond, i.e. no coordination between the amidic carbonyl group to the ruthenium (II) atom and a pentacoordinated environment is existing.

The ligand L is a neutral ligand preferably selected from 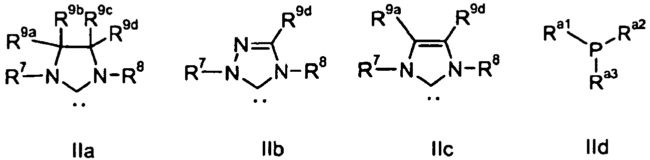 wherein R⁷ and R⁸ independently of each other are C₁₋₆-alkyl, aryl, C₂₋₆- alkenyl or 1-adamantyl and
R^{9a-d} are independently of each other hydrogen, C₁₋₆-alkyl, C₂₋₆- alkenyl or aryl, or R^{9b} and R^{9c} or R^{9a} and R^{9d} taken together form a-(CH₂)₄-bridge;
or R^{9a} and R^{9d} in formula IIc both have the meaning of halogen, preferably of chlorine;
R^{a1}, R^{a2} and R^{a3} independently of each other are C₁₋₆-alkyl, C₃₋₈-cycloalkyl, aryl, heteroaryl or R^{a1} and R^{a2} or R^{a2} and R^{a3} or R^{a1} and R^{a3} taken together form a 1,5-bridged cyclooctyl group.

In a preferred embodiment R⁷ and R⁸ are C₁₋₆-alkyl, 1-adamantyl, phenyl group which is di- or tri-substituted with C₁₋₆-alkyl or naphthyl which is di- or tri-substituted with C₁₋₆-alkyl.

R⁷ and R⁸ more preferably have the meaning of t-butyl, 1-adamantyl, isopropyl, 2, 6-diisopropylphenyl, 2,7-diisopropylnaphthyl or 2, 4, 6-trimethylphenyl, most preferably 2, 4, 6-trimethylphenyl or 2,7-diisopropylnaphthyl.

In a preferred embodiment R^{9a} and R^{9c} are methyl or phenyl and R^{9b} and R^{9d} are hydrogen, or R^{9a} and R^{9c} or R^{9b} and R^{9d} are taken together to form a -(CH₂)ₙ- bridge with n having the meaning of 5 or 6. Its herby understood that if chiral carbon atoms are present, both the racemic and the enantiomerically pure form are comprised.

In a further preferred embodiment R^{9a-d} is hydrogen.

In a preferred embodiment R^{a1}, R^{a2} and R^{a3} independently of each other are C₁₋₆-alkyl, C₃₋₈-cycloalkyl or phenyl.

In a more preferred embodiment R^{a1}, R^{a2} and R^{a3} independently of each other stand for cyclohexyl, cyclopentyl, isopropyl and phenyl.

Suitable representatives of ligands L of formula IId are Cy₃P, iPr₃P, Cyp₃P or Ph₃P wherein Cy stands for cyclohexyl, Cyp for cyclopentyl and iPr for isopropyl.

In a further preferred embodiment L is 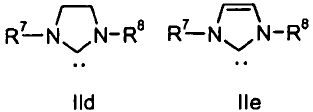 wherein R⁷ and R⁸ are as described above.

As anionic ligand X¹ and X² a halogenide or a pseudo halogenide such as cyanide, a rhodanide, a cyanate, an isocycanate, acetate or trifluoroacetate may be selected. Preferred anionic ligand for X¹ and X² is a halogenide, whereas chloro is the most preferred anionic ligand.

Y¹ is preferably hydrogen.

The preferred meaning of a, b and d is hydrogen.

The preferred meaning for c is hydrogen, halogen, nitro, C₁₋₆-alkylcarbonyl amino, aryl carbonyl amino, aryl sulfonyl amino, alkyl sulfonyl amino, halogen-C₁₋₆-alkyl sulfonyl amino, SO₂-NR'R'' wherein R' and R" independently of each other have the meaning of hydrogen, C₁₋₆-alkyl, aryl or R' and R" together with the N atom form a cycle.

More preferably c means hydrogen, Cl, nitro, SO₂-NR'R''.

In a preferred embodiment R' and R² independently of each other are hydrogen, C₁₋₆-alkyl or

R¹ and R² together with the N atom form a 6 member cycle which contains oxygen as additional hetero atom.

Still more preferred R¹ and R² independently of each other are hydrogen or C₁₋₆-alkyl.

R^{3'} and R^{3''} independently of each other preferably are hydrogen or C₁₋₆-alkyl, more preferably hydrogen or methyl.

The following compounds represent preferred representatives of the present invention.

| | | |
|---|---|---|
| D | 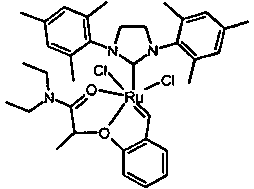 | [RuCl₂(=CH(o-OCH(Me)CONEt₂)Ph)(ImH₂Mes)] |
| E | 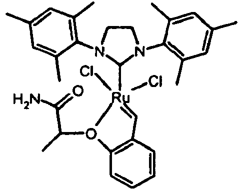 | [RuCl₂(=CH(o-OCH(Me)CONH₂)Ph)(ImH₂Mes)] |
| F | 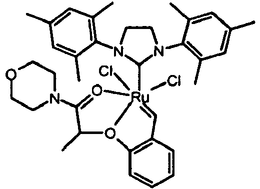 | [RuCl₂(=CH(o-OCH(Me)CO-N-Morpholine)Ph)(ImH₂Mes)] |
| G | 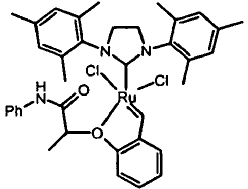 | [RuCl₂(=CH(o-OCH(Me)CONHPh)Ph)(ImH₂Mes)] |
| J | 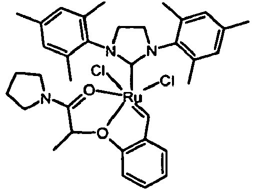 | [RuCl₂(=CH(o-OCH(Me)CO-*N*-Pyrrolidine)Ph)(ImH₂Mes)] |
| K | 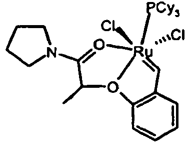 | [RuCl₂(=CH(o-OCH(Me)CO-*N*-Pyrrolidine)Ph)(PCy₃)] |
| L | 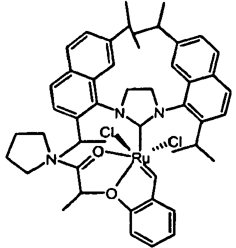 | (RuCl₂(=CH(o-OCH(Me)CO-*N*-Pyrrolidine)Ph)(SIPrNap)] |
| M | 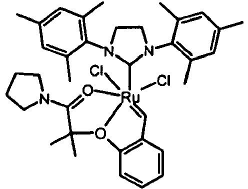 | (RuCl₂(=CH(o-OCMe₂CO-*N*-Pyrrolidine)Ph)(ImH₂Mes)] |
| N | 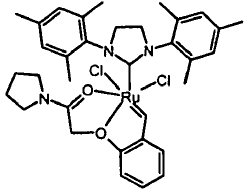 | [RuCl₂(=CH(o-OCH₂CO-*N*-Pyrrolidine)Ph)(ImH₂Mes)] |

Even more preferred are the hexacoordinated Ru (II) complexes of formula D, F, J, L, M and N.

The new compounds of formula I can be obtained by reacting pre-ligands of formula 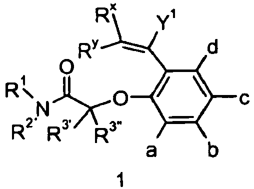 with ruthenium complexes of formula 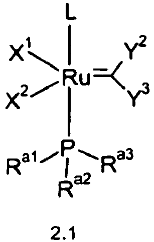 or by reacting pre-ligands of formula **1** with ruthenium complexes of formula 2.2 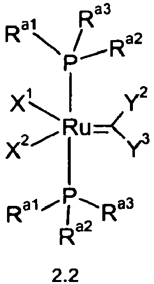 via intermediate ruthenium complexes of formula 2.3 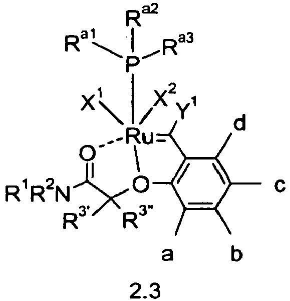 which then can be converted into the compounds of formula **I** by treatment with ligands L of types IIa to IIc.

In the process outlined above the substituents L, X¹, X², Y¹, a,b,c,d, R¹, R², R^{3'} and R^{3''} are as defined above;
Y² and Y³ independently of each other are hydrogen, C₁₋₆-alkyl, C₂₋₆-alkenyl, C₂₋₆-alkynyl, C₁₋₆-alkylthio, aryl, arylthio, C₁₋₆-alkylsulfonyl, C₁₋₆-alkylsulfinyl,
or Y² and Y³ taken together form a cycle of the type 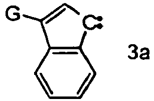 with G being hydrogen or aryl;
or
Y² and Y³ together form a cumulenyl group of type 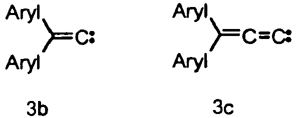 R^{x} and R^{y} independently of each denote hydrogen, C₁₋₆-alkyl optionally substituted by one or more halogen atoms or aryl optionally substituted by one or more halogen atoms or by C₁₋₆-alkyl;
R^{a1}, R^{a2} and R^{a3} independently of each other are C₁₋₆-alkyl, C₃₋₈-cycloalkyl, aryl, heteroaryl or R^{a1} and R^{a2} or R^{a2} and R^{a3} or R^{a1} and R^{a3} form together a 1,5-bridged cyclooctyl group.

The reaction of the pre-ligands of formula **1** with ruthenium complexes of formula **2.1** or **2.2** can in principle be performed following the disclosure in PCT Publication WO 2005/0016944.

Brady et al. J.Org.Chem. 1987, 52, 2216-2220 discloses amides and their use in the intramolecular [2 + 2] cycloaddition of ketene iminium salts. These amides have the potential for being used as preligands in the synthesis of compounds of formula I.

The preligands of formula I 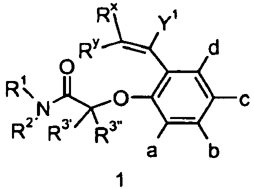 selected from 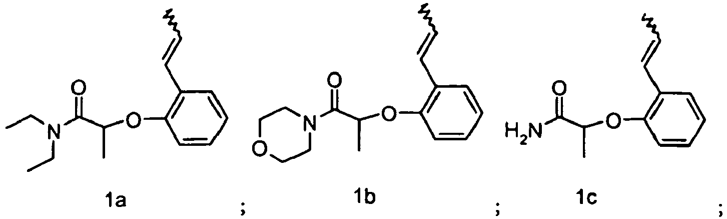 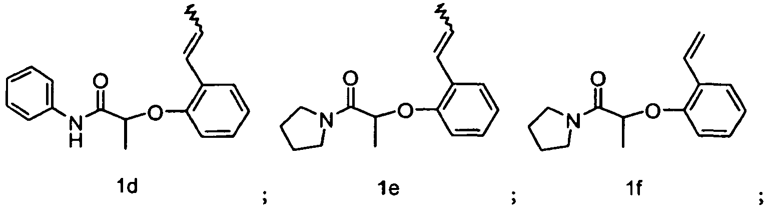 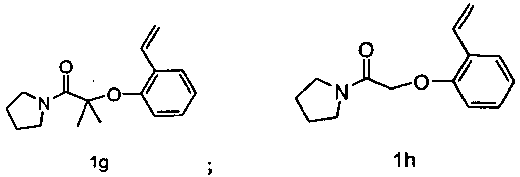 are novel and thus represent a further embodiment of the present invention.

The ligand and complexes can occur as pure enantiomers or mixture of enantiomers. Suitable solvent for the reaction of **1** with **2** is an inert solvent such as a halogenated hydrocarbon like dichloromethane.

The reaction temperature can be chosen between 0°C and 80°C.

For the conversions with preligands of formula 1a to 1e it can be advantageous to add CuCl to the reaction mixture. In this case the reactants are used in equivalent amounts but it can be advantageous to increase the amount of one of the reactants in order to increase the yield.

The compounds of formula I may be separated from other reaction products e.g. by filtration and can be obtained pure by chromatography or crystallization. It is also possible to use the crude products or the catalyst produced in situ directly to carry out the (ring closing) metathesis reactions.

The compounds of formula I can advantageously be used in metathesis reactions, particularly in ring closing metathesis or cross metathesis reactions.

For the determination of the hexacoordination crystals of complex D suitable for X-ray crystal structure analysis were grown by vapor diffusion of pentane into a saturated tetrahydrofuran solution at room temperature.

### Fig. 1

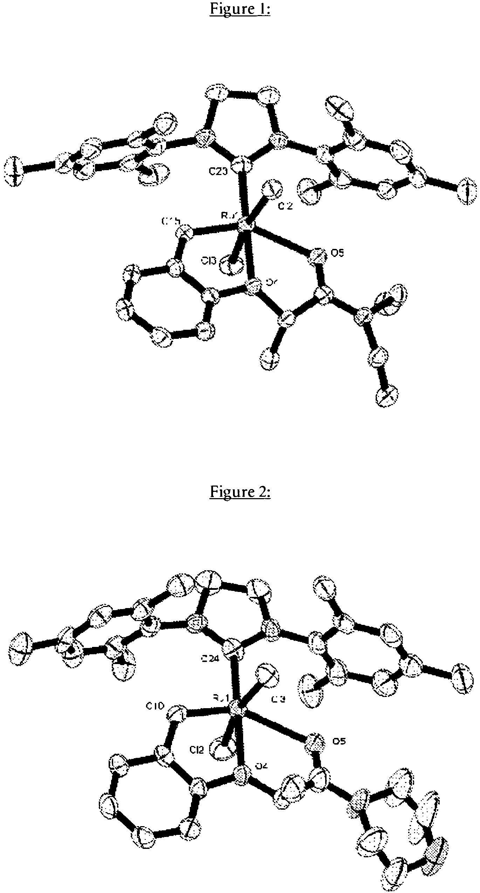

Fig. 1 shows a labeled view of the complex of the formula D

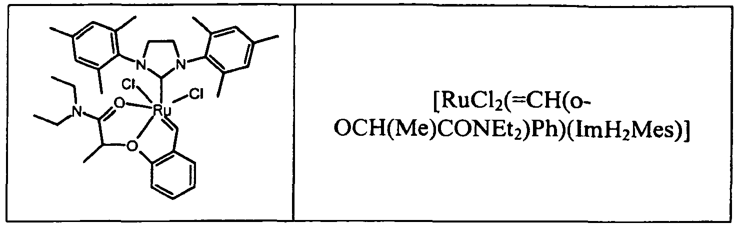

The collection and the refinement of parameters for the crystallographic analysis are summarized in Table X1 and representative bond lengths and bond angles are reported in Table X2 in the examples section.

For the determination of the hexacoordination crystals of complex F suitable for X-ray crystal structure analysis were grown by vapor diffusion of pentane into a saturated dichloromethane solution at room temperature.

### Fig. 2

Fig. 2 shows a labeled view of the complex of the formula F

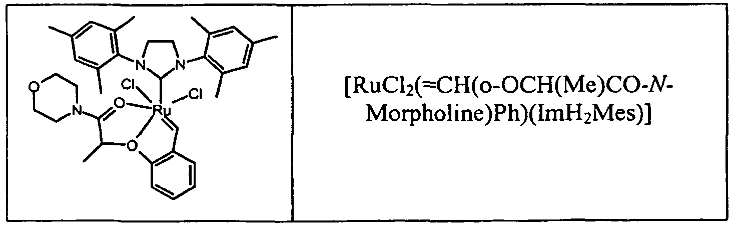

The collection and the refinement of parameters for the crystallographic analysis are summarized in Table X3 and representative bond lengths and bond angles are reported in Table X4 in the examples section.

For the determination of the pentacoordination crystals of complex E suitable for X-ray crystal structure analysis were grown by vapor diffusion of pentane into a saturated tetrahydrofuran solution at room temperature

### Fig. 3

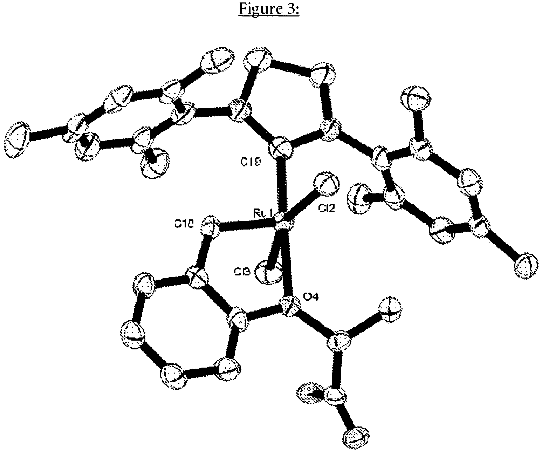

Fig. 3 shows a labeled view of the complex of the formula E

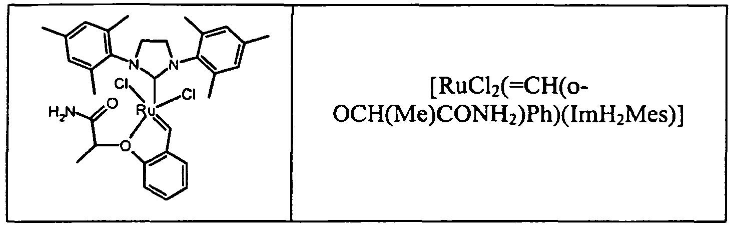

The collection and the refinement of parameters for the crystallographic analysis are summarized in Table X5 and representative bond lengths and bond angles are reported in Table X6 in the examples section.

### Step a)

Step a) requires the transformation of the diene compound of formula IV via RCM reaction into the macrocyclic ester of formula V.
The RCM reaction is as outlined below performed with a compound of the formula I as defined before.

Ppreferred are the following representatives of formula I.

| | | |
|---|---|---|
| D | 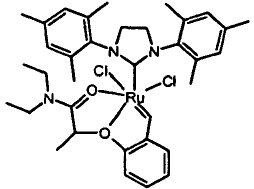 | [RuC]₂(=CH(o-OCH(Me)CONEt₂)Ph)(ImH₂Mes)] |
| E | 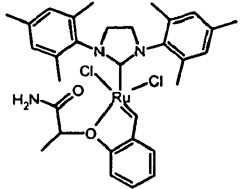 | [RuCl₂(=CH(o-OCH(Me)CONH₂)Ph)(ImH₂Mes)] |
| F | 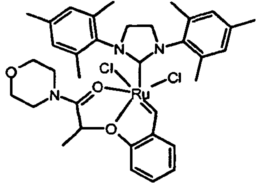 | [RuCl₂(=CH(o-OCH(Me)CO-*N*-Morpholine)Ph)(ImH₂Mes)] |
| G | 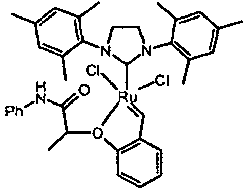 | [RuCl₂(=CH(o-OCH(Me)CONHPh)Ph)(ImH₂Mes)] |
| J | 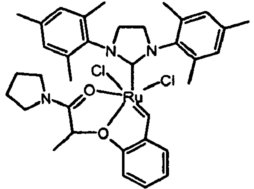 | [RuCl₂(=CH(o-OCH(Me)CO-*N*-Pyrrolidine)Ph)(ImH₂Mes)] |
| K | 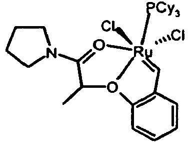 | [RuCl₂(=CH(o-OCH(Me)CO-*N*-Pyrrolidine)Ph)(PCy₃)] |
| L | 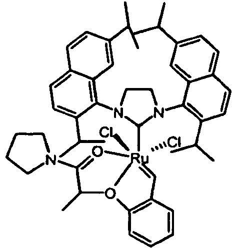 | [RuCl₂(=CH(o-OCH(Me)CO-*N*-Pyrrolidine)Ph)(SIPrNap)] |
| M | 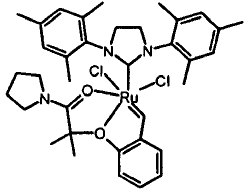 | [RuCl₂(=CH(o-OCMe₂CO-*N*-Pyrrolidine)Ph)(ImH₂Mes)] |
| N | 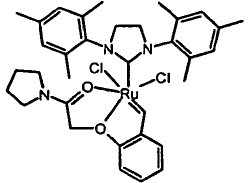 | [RuCl₂(=CH(o-OCH₂CO-*N*-Pyrrolidine)Ph)(ImH₂Mes)] |

Hexacoordinated Ru(II) complexes of formula D, F, J, M, L and N are most preferred.

The RCM reaction is usually performed in an organic solvent, preferably in an aromatic organic solvent such as in benzene, toluene or mesitylene or in halogenated aromatic solvents such as in polyfluorinated benzenes or toluenes. Also halogenated hydrocarbons such as dichloromethane or dichloroethane are suitable solvents. The solvents may be used as single solvent or as a mixture of different solvents. In addition a co-solvent selected from an aliphatic hydrocarbon such as pentane, hexane or heptane may be used as well.

The reaction temperature is as a rule selected in a range of 20°C to 140°C, preferably 40°C to 100°C and even more preferred 50°C to 90°C.

The molar substrate to catalyst ratio S/C is usually selected in a range of 20 to 10000, but preferably in a range of 200 to 4000.

It is convenient to run the reaction either under bubbling of an inert gas through the reaction mixture or under a slight vacuum.

The macrocyclic ester of formula I can be isolated by applying methods known to the skilled in the art such as by column chromatography or by cristallisation. The metathesis reaction mixture can also, after a simple extractive work-up, be brought directly into the next step.

In order to remove most catalyst from the solution of the macrocyclic ester it is convenient to treat the reaction mixture with a complexing agent such as ethylenediamine and to extract the resulting soluble ruthenium species into acidic water. The amount of ethylenediamine is not critical; it can be used in a 1:1 to 100:1 molar ratio relative to the catalyst, preferentially in 20:1 to 70:1 molar ratio.

### Step b)

Step b requires the hydrolysis of the macrocyclic ester of formula V into the macrocyclic acid of formula VI.

In a preferred embodiment the macrocyclic ester of the formula 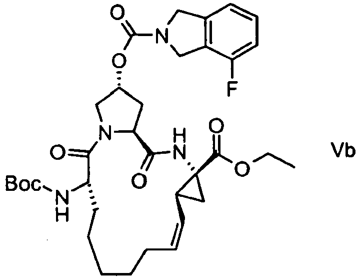 is used.

The hydrolysis can usually be accomplished by treatment with an aqueous alkali hydroxide solution such as with an aqueous sodium hydroxide solution in solvents like methanol or ethanol at a temperature of 0°C to 40°C.

After neutralization of the reaction mixture, usually with hydrochloric acid, the macrocyclic acid of formula VI can be isolated by way of extraction with a suitable solvent such as with dichloromethane. Crystallization in a suitable solvent, preferably in tetrahydrofuran leads to a crystalline product with a purity of over 98 %.

### Step c)

Step c requires the coupling of the macrocyclic acid of formula VI with cyclopropyl sulfonamide to form the macrocyclic sulfonamide of formula VII.

In a preferred embodiment the macrocyclic acid of the formula 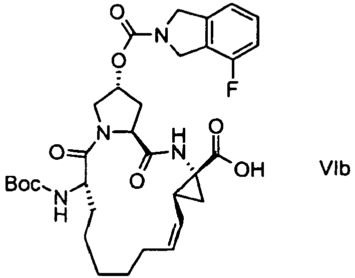 is used.

In a first step the macrocyclic acid of formula VI is reacted with acetic acid anhydride in the presence of an inorganic base, such as with an alkali carbonate like sodium carbonate and a suitable organic solvent such as with tetrahydrofuran into an azlactone intermediate of the formula 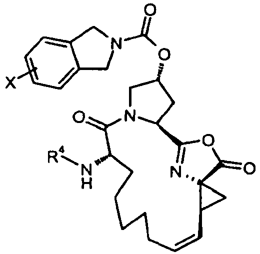 wherein R⁴ is an amino protecting group and X is halogen.

The reaction is expediently performed at a temperature of 10 °C to 50 °C.

As a rule the azlacton intermediate will not be isolated but in situ further reacted with cyclopropyl sulfonamide in the presence of an inorganic base, such as with an alkali carbonate like potassium carbonate to the macrocyclic sulfonamide of formula VII.

The reaction in this second step is expediently performed at a temperature of 50°C to 70°C.

Upon completion of the reaction the reaction mixture can be treated with water. After separation and removal of the water phase the organic phase may further be diluted with a suitable organic solvent such as with ethyl acetate or toluene and washed e.g. with an aqueous sulphuric acid and water.

Isolation of the macrocyclic sulfonamide of formula VII can then be accomplished by a solvent switch to ethanol followed by addition of the ethanolic solution to water thereby causing precipitation of the desired product.

However, in a preferred embodiment the macrocyclic sulfonamide of formula VII will not be isolated, but the organic phase which has been treated as hereinbefore described will be freed of residual water by way of a continuous azeotropic distillation.

The mixture can then directly be used for subsequent step d).

### Step d)

Step d requires the treatment of the macrocyclic sulfonamide of formula VII with a sodium base to form the end product, i.e. the macrocyclic compound of formula III.

In a preferred embodiment the macrocyclic sulfonamide of the formula 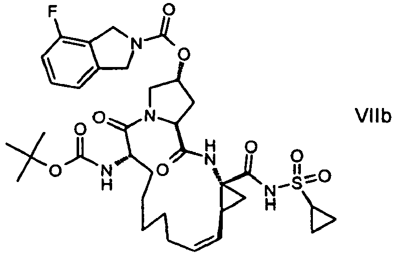 is used.

As a rule the water free mixture obtained from step c) is treated with a sodium base sodium hydroxide, preferably an aqueous solution thereof, sodium methylate or sodium ethoxide, preferably with sodium methylate in the presence of methanol at a temperature of 0°C and 50°C.

Upon completion of the reaction the reaction mixture can be treated with a mixture of a suitable organic solvent such as ethyl acetate and water where after the crystals of the sodium compound of formula III, preferably the compound of formula VIII can be collected in good purity and yield.
The following examples shall illustrate the invention without limiting it.

### Examples

### Abbreviations:

r.t. = room temperature
ImH₂Mes = 1,3-bis-(2,4,6-trimethylphenyl)-2-imidazolidinylidene
ImMes = 1,3-bis-(2,4,6-trimethylphenyl)-2-imidazolylidene
ImH₂Pr = 1,3-bis-(2,6-diisopropylphenyl)-2-imidazolidinylidene
SIPrNap = 1,3-Bis(2,7-diisopropylnaphthalen-1-yl)-2-imidazolidinylidene
RCM = ring closing metathesis
RP column = reverse phase column
S/C = molar substrate-to-catalyst ratio
Mes = 2,4,6-trimethylphenyl
Cy = cyclohexyl
Cyp = cyclopentyl

Diene IVb = 4-Fluoro-1,3-dihydro-isoindole-2-carboxylic acid (3*R*,5*S*)-1-((*S*)-2-tert-butoxycarbonylamino-non-8-enoyl)-5-((1*R*,2*S*)-1-ethoxycarbonyl-2-vinyl-cyclopropylcarbamoyl)-pyrrolidin-3-yl ester of the formula

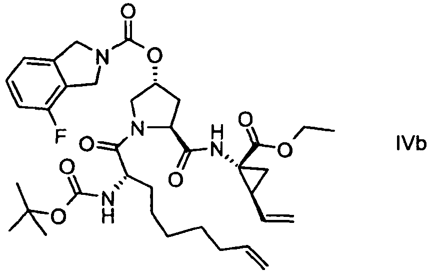

RCM-Ester Vb= (2R,6S,12Z,13aS,14aR,16aS)-Cyclopropa[e]pyrrolo[1,2-a][1,4]diazacyclopentadecine-14a(5H)-carboxylic acid, 6-[[(tert-butoxy)carbonyl]amino]-2-[[(4-fluoro-1,3-dihydro-2H-isoindol-2-yl)carbonyl]oxy]-1,2,3,6,7,8,9,10,11,13a,14,15,16,16a-hexadecahydro-5,16-dioxo-, ethyl ester

The atom numbering is as shown below:

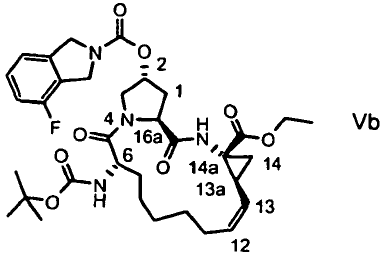

Epi-Vb = 13aR epimer of RCM ester of formula Vb
Epi-IVb: epimer at the vinyl substituted carbon atom of cyclopropyl unit in IVb a% = HPLC area%

| Catalyst Nr. | Catalyst Structure | Chemical Short Name |
|---|---|---|
| A For Comparison | 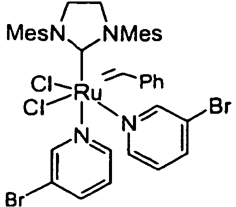 | [RuCl₂(=CHPh)(ImH₂Mes)(m-Br-Pyr)₂] CAS No. 477218-66-9; a) |
| B For Comparison | 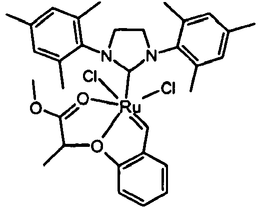 | [RuCl₂(=CH(o-OCH(Me)CO₂Me)Ph)(ImH₂Mes)] CAS No. 837392-94-6 |
| | | M. Bieniek, R. Bujok, M. Cabaj, N. Lugan, G. Lavigne, D. Arlt, K. Grela, J. Am. Chem. Soc. 2006, 128, 13652. |
| C For Comparison | 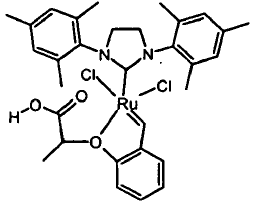 | [RuCl₂(=CH(o-OCH(Me)CO₂H)Ph)(ImH₂Mes)] CAS No. 959710-27-1 |
| | | Generated in situ according to: R. Gawin, A. Makal, K. Wozniak, M. Mauduit, K. Grela, Angew. Chem. Int. Ed. 2007, 46, 7206. |
| D | 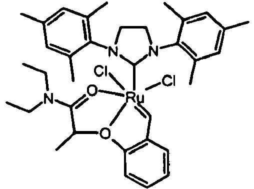 | [RuCl₂(=CH(o-OCH(Me)CONEt₂)Ph)(ImH₂Mes)] |
| E | 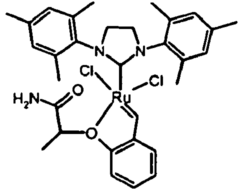 | [RuCl₂(=CH(o-OCH(Me)CONH₂)Ph)(ImH₂Mes)] |
| F | 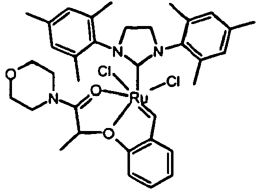 | [RuCl₂(=CH(o-OCH(Me)CO-N-Morpholine)Ph)(ImH₂Mes)] |
| G | 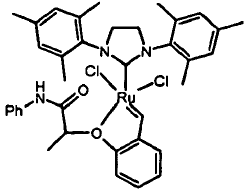 | [RuCl₂(=CH(o-OCH(Me)CONHPh)Ph)(ImH₂Mes)] |
| H For Comparison | 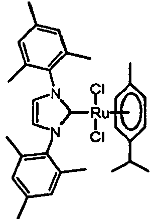 | [RuCl₂(ImMes)(p-cymene)] CAS NO. 244187-82-4 |
| | | L. Jafarpour, J. Huang, E. D. Stevens, S. Nolan, Organometallics 1999, 18, 3760. |
| J | 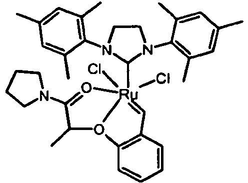 | [RuCl₂(=CH(o-OCH(Me)CO-*N* Pyrrolidine)Ph)(ImH₂Mes)] |
| K | 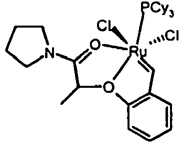 | [RuCl₂(=CH(o-OCH(Me)CO-N-Pyrrolidine)Ph)PCy₃)] |
| L | 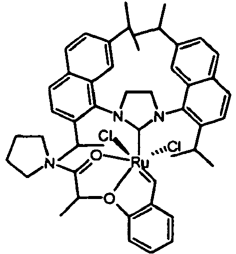 | [RuCl₂(=CH(o-OCH(Me)CO-*N*-Pyrrolidine)Ph)(SIPrNap)] |
| M | 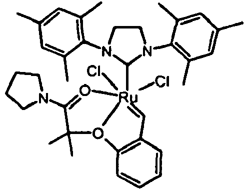 | [RuCl₂(=CH(o-OCMe₂CO-*N*-Pyrrolidine)Ph)(ImH₂Mes)] |
| N | 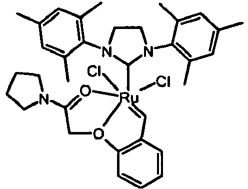 | [RuCl₂(=CH(o-OCH₂CO-*N*-Pyrrolidine)Ph)(ImH₂Mes)] |

| | | |
|---|---|---|
| a) Commercially available from Sigma-Aldrich Chemie GmbH, Postfach, CH-9471 Buchs, Switzerland. | | |

### Example 1

In a glove-box (O₂ < 2 ppm) a solution of 50.0 mg (0.073 mmol, corrected by content) of diene IVb and 2.62 mg (0.0036 mmol) of catalyst D in 6.5 ml of toluene (distilled under argon) was stirred at 65°C in a 15 ml screw-capped flask. After 4 h one drop of ethylene diamine was added and the mixture was stirred for 10 min outside of the glove box. After addition of 1 ml of 1 M aqueous solution of hydrochloric acid the biphasic mixture was stirred for 10 min. A 0.5 ml aliquote of the organic phase was removed and evaporated to dryness; the oily residue was dissolved in 1 ml of acetonitrile and analyzed by HPLC. Conversion was >99 area%, the desired product (RCM-ester Vb) had 67 area% purity.

HPLC method on reverse phase (RP) column: Waters XBridge C18 column, 4.6 x 150 mm, solvent A: water/acetonitrile 95/5, solvent B: acetonitrile, gradient from A/B 50/50 to 10/90 within 11 min, then 4 min at 10/90, 40°C, 210 nm, 1 ml/min. Retention times: toluene 5.2 min, diene IVb 8.85 min, RCM-ester Ib 6.97 min (identified by HPLC/MS, [MH]⁺ 657.4 u), peaks of dimeric by-products at 10.2, 10.4,12.1 and 13.1 min (MS: [MH]⁺ 1313 u). Only the sum of the dimer peaks is given in the tables and experiments.

HPLC method on chiral column: Chiralcel OD-RH, 4.6-150 mm, solvent A: water + 5% acetonitrile (62%), acetonitrile (38%), no gradient, 40°C, 1 ml/min, 210 nm. Retention times: diene IVb 3.4 min, 2R epimeric diene epi-IVb74.2 min, RCM ester Vb 47.6 min, at 13a epimeric RCM-ester V (EpiVb) 33.9 min.

### Examples 2a-2i

The examples in Table 1 were carried out using the same procedure and conditions (if not specifically mentioned in the footnote) as in Example 1 but in the presence of various catalysts.

**Table 1**

| | | RP column | | | chiral column | |
|---|---|---|---|---|---|---|
| Reaction Nr. | Catalyst Nr. | Diene IVb (a %) | RCM-ester Vb (a %) | Dimers (a %) | RCM ester Vb (a %) | Vb/epi-Vb |
| 2a | A | 6 | 74 | 11 | 74 | 99/1 |
| 2b | C | 0.2 | 78 | 16 | 82 | 99/1 |
| 2c | B | 2 | 58 | 13 | 62 | 77/23 |
| 2d | E | 1 | 72 | 18 | 79 | 99/1 |
| 2e | F | 0.3 | 67 | 14 | 88 | 89/11 |
| 2f | G | 0.3 | 77 | 16 | 99 | >99/1 |
| 2g | H | 31 | 37 | 3 | 48 | 84/16 |
| 2h | J | 0.2 | 64 | 13 | 85 | 86/14 |
| 2i | K | 12 | 71 | 8 | 85 | >99/1 |
| 2j | L | <0.1 | 70 | 12 | 96 | 98/2 |
| 2k | M | 0.5 | 69 | 12 | 88 | 90/10 |
| 2l | N | 0.2 | 76 | 11 | 96 | 97/3 |

| | | | | | | |
|---|---|---|---|---|---|---|
| %y. = % yield determined by HPLC with internal standard; a%: HPLC area%; n.d.: not determined. Reaction Nr. 2g was run at 80°C under addition of 0.007 mmol of phenylacetylene. | | | | | | |

### Example 3 (S/C 135-200)

To a solution of 2.67 g (2.00 mmol) of diene IVb (as a 51.4% solution in toluene) in 155 ml of toluene was added under argon bubbling (33 ml/min) at 60 °C 6.58 mg (0.005 mmol) of catalyst E. After 2 h stirring at this temperature (an analytical sample was taken, if required) 3.26 mg of catalyst E were added. After a total of 5 h 50 µl (0.74 mmol) of ethylenediamine were added and the mixture was stirred at room temperature for 10 min. After this time the mixture was extracted with 1 M aqueous solution of hydrochloric acid and with water. Evaporation of the organic phase afforded 1.32 g of RCM-ester Vb with 78.5% purity.

### Examples 4a-4f (S/C 135-200)

The examples in Table 2 were carried out using the same procedure and conditions as in Example 3, but in the presence of various catalysts.

**Table 2**

| | | RP column | | | chiral column | |
|---|---|---|---|---|---|---|
| Reaction Nr. | Catalyst Nr. | Diene IV b a% | RCM-Ester Vb a% | Dimers a% | RCM-Ester Vb a % | Epi-Vb a% |
| 4a | D | 23 | 59 | 5 | >99.8 | <0.2 |
| 4b | B | 5 | 76 | 12 | >99.8 | <0.2 |
| 4c | C | 0.5 | 80 | 14.6 | >99.8 | <0.2 |
| 4d | F | 28 | 55 | 4 | 68 | <0.2 |
| 4e | G | 2.7 | 78 | 13 | >99.8 | <0.2 |
| 4f | J | 18 | 64 | 6 | >99.8 | <0.2 |
| 4g | J | 26 | 57 | 10 | >99.8 | <0.2 |
| 4h | N | 10 | 72 | 11 | >99.8 | <0.2 |
| 4i^{*)} | L | 1.7 | 79 | 13 | >99.8 | <0.2 |
| 4j | M | 27 | 52 | 10 | >99.8 | <0.2 |

| | | | | | | |
|---|---|---|---|---|---|---|
| %y. = % yield determined by HPLC with internal standard; a%: HPLC area%; Reactions Nr. 4a to 4f have been carried out with crude diene IVb at 60°C; reactions 4g to 4j have been carried out at 50°C with diene IVb which had been purified by chromatography on silicagel and crystallization from toluene/diethyl ether. ^{*)} No catalyst added after 2h. | | | | | | |

### Example 5 (S/C 1000, vacuum, P= 0.26 bar)

To a solution of 6.60 g (5.00 mmol) of diene IVb (as a 51.4% solution in toluene) in 390 ml of toluene was added at 70°C under vacuum (pressure = ca. 0.26 bar) by dropping funnel a solution of 3.59 mg (0.005 mmol) of catalyst D in 20 ml of toluene. The catalyst was added during ca. 1 h. Under these conditions a small amount of toluene (19 ml) distilled off in the course of the reaction. After 2 h of total reaction time 17 µl (0.252 mmol) of ethylene diamine were added at ambient pressure, the reaction mixture was concentrated under vacuum, washed with 0.5 M aqueous solution of hydrochloric acid, treated with decolorizing charcoal and evaporated to dryness. RCM-ester Vb was isolated as a off-white solid (3.58 g) with 84.2 a% purity (75.7% content, 82.5% yield).

### Examples 6a-f

The experiments in Table 3 have been carried out in analogy to Example 5, Catalyst Nr., temperature, reaction time, yield and purity of RCM ester Vb are given in the table.

**Table 3**

| | | | RP column | | | chiral column | |
|---|---|---|---|---|---|---|---|
| Reaction Nr. | Catalyst Nr. | T °C | Diene IVb a% | RCM-Ester Vb a %/ %y. | Dimers a% | RCM-Ester Vb a % | Epi-Vb a% |
| 6a | B | 70 | 0.2 | 84/83 | 12.2 | >99.8 | <0.2 |
| 6b | C | 70 | 1.6 | 84/81 | 10.0 | 99.8 | 0.2 |
| 6c | E | 70 | 1.4 | 83/82 | 10.3 | >99.8 | <0.2 |
| 6d | F | 70 | 0.3 | 83/85 | 13.4 | >99.8 | <0.2 |
| 6e^{$} | G | 70 | 1.7 | 83/82 | 10 | >99.8 | <0.2 |
| 6f | J | 70 | 0.3 | 84/- | 11 | >99.8 | <0.2 |

| | | | | | | | |
|---|---|---|---|---|---|---|---|
| %y. = % yield determined by HPLC with internal standard; a%: HPLC area% ^{$} Total reaction time was 4 h. | | | | | | | |

### Examples 7a-e

The experiments in Table 4 have been carried out in analogy to example 5, but with following changes: 10 mmol of diene IVb in 780 ml of toluene, catalyst (type and amount cf. Table 4) added during ca. 1.5 h as a solution in 40 ml of toluene, temperature 70°C.

**Table 4**

| | | | RP column | | | chiral column | |
|---|---|---|---|---|---|---|---|
| Reaction Nr. | Catalyst Nr. | S/C | Diene IVb a% | RCM-Ester Vb a%/ %y. | Dimers a% | RCM-Ester Vb a % | Epi-Vb a% |
| 7a | D | 2000 | 1.7 | 83/83 | 10 | >99.8 | <0.2 |
| 7b^{$} | D | 2500 | 4.3 | 82/ 79 | 9 | >99.8 | <0.2 |
| 7c^{#} | E | 2000 | 2.5 | 83/80 | 9 | >99.8 | <0.2 |
| 7d^{£} | E | 2200 | 4.5 | 79/ - | 10 | >99.8 | <0.2 |
| 7e | F | 2200 | 1.2 | 84/83 | 10 | >99.8 | <0.2 |
| 7f | L | 2000 | 4.7 | 83/82 | 9 | >99.8 | <0.2 |

| | | | | | | | |
|---|---|---|---|---|---|---|---|
| %y. = % yield determined by HPLC with internal standard; a%: HPLC area% ^{$} Total reaction time was 2.5 h, the catalyst was added during 1.5 h. ^{#} Total reaction time was 4 h. ^{£} Total reaction time was 5 h. | | | | | | | |

### Example 8

### 2-[((E,Z)-2-propenyl)-phenoxy]-propionamide

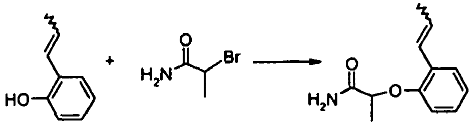

To a suspension of 12.24 g (87.6 mmol) of potassium carbonate and 5.78 g (17.7 mmol) of cesium carbonate in 200 ml of *N,N*-dimethylformamide, 6.00 g (43.8 mmol) of (*E*,*Z*)-2-propenylphenol (4:1 mixture of *E*/*Z*-isomers) was added. After stirring for 30 min at room temperature, 6.73 g (43.8 mmol) of 2-bromo-propionamide was added and the reaction mixture was stirred for 2 days at 40°C. The reaction mixture was filtered and concentrated at 50°C/10 bar). To the residue, 150 ml of diethyl ether and 150 ml of water were added. The layers were separated and the aqueous layer extracted with 200 ml of diethyl ether. The combined organic layers were washed successively with 100 ml of water and 100 ml of brine, dried over sodium sulfate and evaporated to dryness at 40°C/10 mbar to yield 9.44 g (96%) of the title compound as a 3.5:1 mixture of *E*/*Z*-isomers with 91% purity (GC-area%) as white crystals. (GC method: Column HP-5, 5% phenyl methyl siloxane, 30 m x 0.32 mm, df: 0.25 □m; injector temp.: 250°C; detector temp.: 250°C; oven temp.: 50°C to 300° (10°C/min), then 300 °C for 5 min ; retention times: 2-bromo-propionamide 4.8 min, (Z)-2-propenylphenol 6.7 min, (*E*)-2-propenylphenol 8.5 min, 2-[((*Z*)-2-propenyl)-phenoxy]-propionamide at 14.3 min, 2-[((*E*)-2-propenyl)-phenoxyl-propionamide 15.0 min).

MS: 206.0 (M+H⁺).

### Example 9

### 2-[((E,Z)-2-Propenyl)-phenoxy]-propionic acid

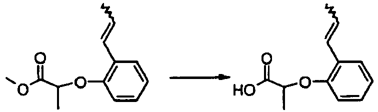

To a solution of 0.50 g (2.2 mmol) of methyl 2-[((*E,Z*)-2-propenyl)-phenoxy]-propanoate

(4:1 mixture of *E*/*Z* isomers, prepared according to D. Arlt, K. Grela et al, J. Am. Chem. Soc. 2006, 128, 13652-13653) in dioxane, 11 ml (20.0 mmol) of a 2M aqueous sodium hydroxide solution was added and the reaction mixture was stirred for 16 h at room temperature. To the reaction mixture, 50 ml of water and 100 ml of tert.-butyl methyl ether were added. The organic layer was washed with 40 ml of water. After the pH of the combined aqueous layers was adjusted with 25% aqueous hydrochloric acid to a value of 1, 150 ml of dichloromethane was added. The organic layer was washed with 100 ml of brine, dried over sodium and evaporated to dryness at 40°C/10 mbar to yield 0.50 g (99% yield) of the title compound as a 3:1 mixture of *E*/*Z*-isomers with >99.9% purity (GC-area%) as white crystals. (GC method as described in Example 7. Retention times: Methyl 2-[((*Z*)-2-propenyl)-phenoxy]-propanoate 12.2 min, methyl 2-[((*E*)-2-propenyl)-phenoxy]-propanoate 12.9 min, 2-[((*Z*)-2-propenyl)-phenoxy]-propionic acid 13.3 min, 2-[((E)-2-propenyl)-phenoxy]-propionic acid 14.0 min).

Mp.: 96°C. MS: 206.0 (M⁺).

### Example 10

### N,N-Diethyl-2-[((E,Z)-2-propenyl-phenoxy]-propionamide

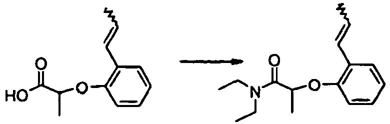

To a solution 1.26 ml (12.1 mmol) of diethylamine in 100 ml of *N,N*-dimethylformamide, 2.12 ml (12.1 mmol) of *N,N*-diisopropylethylamine, 0.50 g (2.4 mmol) of 2-[((*E,Z*)-2-propenyl)-phenoxy]-propionic acid (3:1 mixture of *E*/*Z*-isomers) and 1.01 g (3.0 mmol) of *O*-benzotriazol-1-yl-*N,N,N',N*-tetramethyluronium tetrafluoroborate (TBTU) was added and the reaction mixture stirred for 16 h at room temperature. To the reaction mixture 100 ml of water and 200 ml ethyl acetate were added. The organic layer was separated, washed with 50 ml of water, dried over sodium sulfate and evaporated to dryness at 40°C/10 mbar. The crude title product was purified by silica gel chromatography (heptane/ethyl acetate 3:1) to yield 0.57 g (87% yield) of the title compound as a 4:1 mixture of *E*/*Z*-isomers with 96.2 % purity (GC-area%) as a colorless oil. (GC method as described in Example 7. Retention times: 2-[((Z)-2-propenyl)-phenoxy]-propanoic acid 13.9 min, 2-[((*E*)-2-propenyl)-phenoxy]-propanoic acid 14.0 min, *N,N*-diethyl-2-[((*Z*)-2-propenyl)-phenoxy]-propionamide 16.2 min, *N,N*-diethyl-2-[((*E*)-2-propenyl)-phenoxy]-propionamide 16.6 min).

MS: 262.0 (M+H⁺).

### Example 11

### 1-Morpholine-4-yl-2-[((E,Z)-2-propenyl)-phenoxy]-propan-1-one

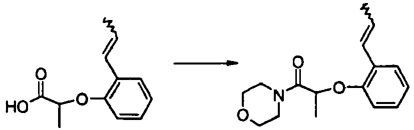

To a solution 0.21 ml (2.4 mmol) of morpholine in 13 ml of *N,N*-dimethylformamide, 0.42 ml (2.4 mmol) of *N,N*-diisopropylethylamine, 0.10 g (0.5 mmol) of 2-[((*E,Z*)-2-propenyl)-phenoxy]-propionic acid (3:1 mixture of *E*/*Z*-isomers) and 0.20 g (0.6 mmol) of *O*-benzotriazol-1-yl-*N,N,N',N*'-tetramethyluronium tetrafluoroborate (TBTU) was added and the reaction mixture stirred for 45 min at room temperature. To the reaction mixture 20 ml of water and 40 ml ethyl acetate were added. The organic layer was separated, washed with 10 ml of water, dried over sodium sulfate and evaporated to dryness at 40°C/10 mbar. The crude title product was purified by silica gel chromatography (heptane/ethyl acetate 3:1) to yield 71 mg (53% yield) of the title compound as a 4:1 mixture of *E*/*Z*-isomers with >99.9 % purity (GC-area%) as a white powder. (GC method as described in Example 7. Retention times: 2-[((2)-2-propenyl)-phenoxy]-propanoic acid 13.9 min, 2-[((*E*)-2-propenyl)-phenoxy]-propanoic acid 14.0 min, 1-morpholine-4-yl-2-[((*Z*)-2-propenyl)-phenoxy]-propan-1-one 18.4 min, 1-morpholine-4-yl-2-[((*E*)-2-propenyl)-phenoxy]-propan-1-one 18.7 min).

MS: 276.1 (M+H⁺).

### Example 12

### N-Phenyl-2-[((E,Z)-2-propenyl)-phenoxy]-propionamide

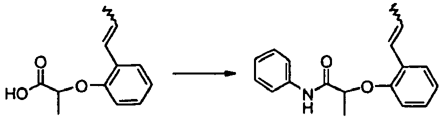

To a solution 0.11 ml (1.2 mmol) of aniline in 6 ml of N,N-dimethylformamide, 0.21 ml (1.2 mmol) of *N,N*-diisopropylethylamine, 0.05 g (0.3 mmol) of 2-[((*E,Z*)-2-propenyl)-phenoxy]-propionic acid (3:1 mixture of *E*/*Z*-isomers) and 0.10 g (0.3 mmol) of *O*-benzotriazol-1-yl-*N,N,N',N*'-tetramethyluronium tetrafluoroborate (TBTU) was added and the reaction mixture stirred for 2 h at room temperature. To the reaction mixture 10 ml of water and 20 ml ethyl acetate were added. The organic layer was separated, washed with 10 ml of water, dried over sodium sulfate and evaporated to dryness at 40°C/10 mbar. The crude title product was purified by silica gel chromatography (heptane/ethyl acetate 3:1) to yield 58 mg (85% yield) of the title compound as a 4:1 mixture of *E*/*Z*-isomers with >99.9 % purity (GC-area%) as a white powder. (GC method as described in Example 7. Retention times: 2-[((*Z*)-2-propenyl)-phenoxy]-propanoic acid 13.9 min, 2-[((*E*)-2-propenyl)-phenoxy]-propanoic acid 14.0 min, *N*-phenyl-2-[((*Z*)-2-propenyl)-phenoxy]-propionamide 19.9 min, *N*-phenyl-2-[((*E*)-2-propenyl)-phenoxy]-propionamide 20.3 min).

MS: 282.3 (M+H⁺).

### Example 13a

### 2-[((E,Z)-2-Propenyl)-phenoxy]-1-pyrrolidine-1-yl-propan-1-one

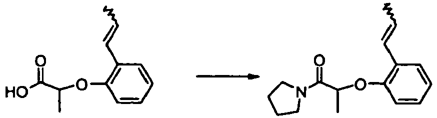

To a solution 1.92 ml (23.0 mmol) of pyrrolidine in 200 ml of *N,N*-dimethylformamide, 4.02 ml (23.0 mmol) of *N,N*-diisopropylethylamine, 1.00 g (4.6 mmol) of 2-[((*E,Z*)-2-propenyl)-phenoxy]-propionic acid (3:1 mixture of *E*/*Z*-isomers) and 1.92 g (5.8 mmol) of *O*-benzotriazol-1-yl-*N,N,N',N*'-tetramethyluronium tetrafluoroborate (TBTU) was added and the reaction mixture stirred for 2 h at room temperature. To the reaction mixture 200 ml of water and 400 ml ethyl acetate were added. The organic layer was separated, washed with 100 ml of water, dried over sodium sulfate and evaporated to dryness at 40°C/10 mbar. The crude title product was purified by silica gel chromatography (heptane/ethyl acetate 3:1) to yield 0.69 g (57% yield) of the title compound as a 4:1 mixture of *E*/*Z*-isomers with 98.1 % purity (GC-area%) as a white powder. (GC method as described in Example 7. Retention times: 2-[((*Z*)-2-propenyl)-phenoxy]-propanoic acid 13.9 min, 2-[((*E*)-2-propenyl)-phenoxy]-propanoic acid 14.0 min, 2-[((*Z*)-2-propenyl)-phenoxy]-1-pyrrolidine-1-yl-propan-1-one 18.0 min, 2-[((*E*)-2-propenyl)-phenoxy]-l-pyrrolidine-1-yl-propan-1-one 18.4 min).

MS: 260.0 (M+H⁺).

### Example 13b

### 1-Pyrrolidin-1-yl-2-(2-vinylphenoxy)-propan-1-one

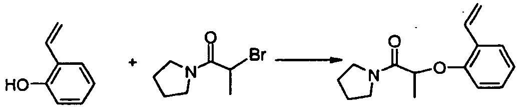

To a solution of 7.00 g (30.47 mmol, 89.4% pure by GC analysis) of 2-bromo-1-pyrrolidin-1-yl-propan-1-one and 3.7 g (30.49 mmol) of 2-hydroxystyrene in 120 ml of *N,N*-dimethyl formamide were added 8.20 g (59.33 mmol) of potassium carbonate and 3.90 g (11.91 mmol) of cesium carbonate. The yellow suspension was stirred at 45°C for 3.5 h. The reaction mixture was filtered and the filtrate was concentrated at 60°C/10 bar. The resulting thick suspension was stirred vigorously in a mixture of ethyl acetate and deionized water. The organic phase was washed again with water, dried with sodium sulfate and evaporated to dryness. Crystallization of the light brown semisolid residue from tert-butyl methylether / heptane and drying (50°C/10 mbar) afforded 5.4 g of the title compound as a white powder with a melting point of 84-85°C.

MS: 246.1496 (M+H)⁺, 268.1317 (M+Na)⁺.

### Example 13c

### 2-Methyl-1-pyrrolidin-1-yl-2-(2-vinylphenoxy)-propan-1-one

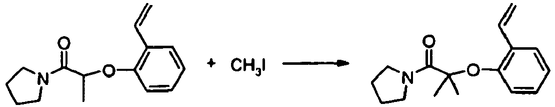

To a solution of lithium diisopropylamide (prepared at -40°C by treatment of 3.492 ml (24.46 mmol) of diisopropylamin with 14.0 ml (22.42 mmol) of a 1.6 molar solution of n-butyllithium in hexane) in 100 ml of tetrahydrofuran was added at -40°C a solution of 5.00 g (20.38 mmol) of 1-pyrrolidin-1-yl-2-(2-vinylphenoxy)-propan-1-one in 20 ml of tetrahydrofuran. The mixture was stirred at the same temperature during 30 min, then 1.55 ml (25.0 mmol) of iodomethane was added dropwise. Addition after 1 h of water, extraction of the organic phase at room temperature with 1 molar HCl solution followed by 1 molar NaOH solution afforded after drying (sodium sulfate) crude product which was purified by column chromatography on silica gel to afford 4.5 g of the title compound as a light yellow oil which became solid on standing.

MS: 260.1 (M+H)⁺, 282.5 (M+Na)⁺.

### Example 13d

### Pyrrolidin-1-yl-2-(2-vinylphenoxy)-ethanone

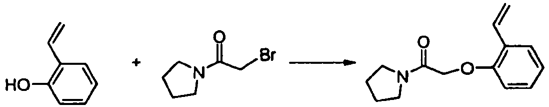

To a solution of 12.7 g (80.2 mmol) of the potassium salt of 2-hydroxystyrene (obtained by treatment of 2-hydroxystyrene with potassium hydroxide in water) in 180 ml of water was added 300 ml of toluene and 1.0 g (3.07 mmol) of tetrabutylammonium bromide. Then, a solution of 16.3 g (81.6 mmol) of 2-bromo-1-(1-pyrrolidinyl)-ethanone in 14 ml of toluene was added and the biphasic mixture was stirred vigorously at 40°C. After 16 hours the reaction mixture was cooled to room temperature, the organic phase was washed with 2 molar NaOH solution, with 1 molar HCl solution, dried over sodium sulfate and evaporated to dryness to afford the title compound with 99.9% purity by GC.

MS: 232.1327 (M+H)⁺, 254.1150 (M+Na)⁺.

### Example 14

### Catalyst No. D, [RuCl₂(=CH(o-OCH(Me)CONEt₂)Ph)(ImH₂Mes)]

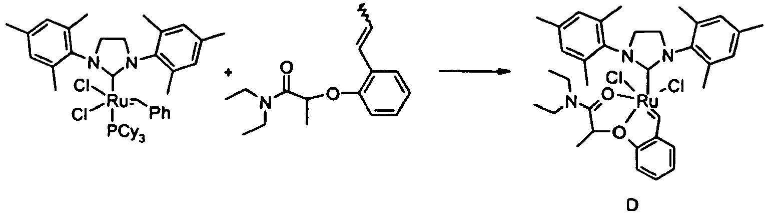

A suspension of 1.50 g (1.77 mmol) of [RuCl₂(PCy₃)(ImH₂Mes)(phenylmethylene)] (commercially available from Sigma-Aldrich Inc., St. Louis, USA), 0.19 g (1.94 mmol) of copper chloride and 0.51 g (1.94 mmol) of *N,N*-diethyl-2-[((*E,Z*)-2-propenyl)-phenoxy]-propionamide as a 4:1 mixture of *E*/*Z* isomers in 110 ml of dichloromethane was stirred for 40 min at 40°C. The reaction mixture was evaporated to dryness at 40°C/10 mbar. The crude title product was purified by repeated digestion with ethylacetate / pentane / tetrahydrofuran to yield 0.73 g (58%) of the title compound as a green crystalline solid.

MS: 711.2 (M⁺). Anal. calcd. for C₃₅H₄₅Cl₂N₃O₂Ru: C, 59.06; H, 6.37; N, 5.90; Cl, 9.96. Found: C, 58.56; H, 6.44; N, 5.23; Cl, 9.86.

Crystals of the title compound suitable for X-ray crystal structure analysis were grown by vapor diffusion of pentane into a solution of 20 mg of [RuCl₂(-CH(o-OCH(Me)CONEt₂)Ph)(ImH₂Mes)] in 2 ml of tetrahydrofuran at room temperature.

Fig. 1 shows a labeled view of the complex of formula D.

**Table X1**

| Crystal data and structure refinement for complex D | |
|---|---|
| Empirical formula | C35 H45 Cl2 N3 02 Ru |
| Formula weight | 711.71 |
| Crystal habit | Green, cubical |
| Temperature | 110 K |
| Wavelength | 0.7107 A |
| Crystal system, space group | Monoclinic, C2/c |
| Unit cell dimensions | a = 30.5023(11) A; alpha = 90 deg. |
| | b = 13.0991(4) A; beta = 102.893(4) deg. |
| | c = 17.1676(5) A; gamma = 90 deg. |
| Volume | 6686.4(4) A³ |
| Z, Calculated density | 8, 1.414 Mg/m³ |
| Absorption coefficient | 0.664 mm⁻¹ |
| F(000) | 2960 |
| Crystal size | 0.18 x 0.18 x 0.15 mm |
| Theta range for data collection | 3.04 to 26.37 deg. |
| Limiting indices | -38<=h<=38, -15<=k<=16, -21<=1<=21 |
| Reflections collected / unique | 24158 / 6073 [R(int) = 0.0362] |
| Completeness to theta = 26.37 | 88.7 % |
| Absorption correction | Semi-empirical from equivalents |
| Max. and min. transmission | 1.00000 and 0.84916 |
| Refinement method | Full-matrix least-squares on F² |
| Data / restraints / parameters | 6073 / 0 / 401 |
| Goodness-of-fit on F² | 0.966 |
| Final R indices [I>2sigma(I)] | R1 = 0.0291, wR2 = 0.0727 |
| R indices (all data) | R1 = 0.0426, wR2 = 0.0764 |
| Largest diff. peak and hole | 0.415 and -0.476 e.A⁻³ |

**Table X2**

| Selected Bond Lengths (Å) and Angles (deg) for Complex D | |
|---|---|
| Bond Lengths (A) | |
| Ru(1) C(15) | 1.840(2) |
| Ru(1) C(23) | 1.984(2) |
| Ru(1) O(4) | 2.1873(16) |
| Ru(1) O(5) | 2.3494(16) |
| Ru(1) Cl(2) | 2.3692(6) |
| Ru(1) Cl(3) | 2.4023(6) |
| | |

| Bond Angles (deg) | |
|---|---|
| C(15)-Ru(1)-C(23) | 99.12(10) |
| C(15)-Ru(1)-O(4) | 80.90(8) |
| C(23)-Ru(1)-O(4) | 173.37(8) |
| C(15)-Ru(1)-O(5) | 147.19(9) |
| C(23)-Ru(1)-O(5) | 112.54(8) |
| O(4)-Ru(1)-O(5) | 68.66(6) |
| C(15)-Ru(1)-Cl(2) | 100.04(8) |
| C(23)-Ru(1)-Cl(2) | 89.05(7) |
| O(4)-Ru(1)-Cl(2) | 84.43(5) |
| O(5)-Ru(1)-Cl(2) | 89.22(4) |
| Cl(2)-Ru(1)-Cl(3) | 170.68(2) |

### Example 15

### Catalyst No. D, [RuCl₂(=CH(o-OCH(Me)CONEt₂)Ph)(ImH₂Mes)]

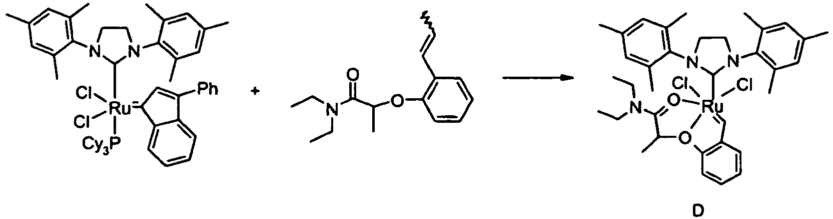

A suspension of 0.50 g (0.53 mmol) of [RuCl₂(PCy₃)(ImH₂Mes)(3-phenyl-indenylidene)] (commercially available from Umicore AG, D-63457 Hanau-Wolfgang), 0.06 g (0.59 mmol) of copper chloride and 0.16 g (0.53 mmol) of *N,N*-diethyl-2-[((*E,Z*)-2-propenyl)-phenoxy]-propionamide as a 4:1 mixture of *E*/*Z*-isomers in 16 ml of dichloromethane was stirred for 40 min at 40°C. The reaction mixture was evaporated to dryness at 40°C/10 mbar. The residue was stirred in 45 ml of tetrahydrofuran for 15 min at room temperature. The dark green suspension was filtered and the filtrate was evaporated to dryness at 40°C/10 mbar. The crude title product was purified by repeated digestion with ethylacetate / pentane to yield 0.29 g (76%) of the title compound as a green crystalline solid.
MS: 711.2 (M⁺).

### Example 16

### Catalyst No. F, [RuCl₂(=CH(o-OCH(Me)CO-N-Morpholine)Ph)(ImH₂Mes)]

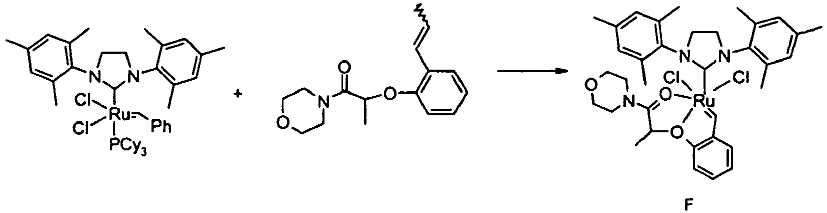

A suspension of 1.00 g (1.18 mmol) of [RuCl₂(PCy₃)(ImH₂Mes)(phenylmethylene)] (commercially available from Sigma-Aldrich Inc., St. Louis, USA), 0.13 g (1.30 mmol) of copper chloride and 0.36 g (1.30 mmol) of 1-morpholine-4-yl-2-[((*E,Z*)-2-propenyl)-phenoxy]-propan-1-one as a 4:1 mixture of *E*/*Z*-isomers in 75 ml of dichloromethane was stirred for 30 min at 40°C. The reaction mixture was evaporated to dryness at 40°C/10 mbar. The residue was stirred in 250 ml of ethyl acetate for 30 min at room temperature. The dark green suspension was filtered and the filtrate was evaporated to dryness at 40°C/10 mbar. The crude title product was purified by silica gel chromatography (cyclo-hexane/ethyl acetate 1:2) to yield 0.38 g (45% yield) of the title compound as a green powder.

MS: 725.2 (M⁺). Anal. calcd. for C₃₅H₄₃Cl₂N₃O₃Ru · ½ CH₂Cl₂: C, 55.51; H, 5.77; N, 5.47; Cl, 13.85. Found: C, 54.75; H, 5.76; N, 5.30; Cl, 13.71.

Crystals of the title compound suitable for X-ray crystal structure analysis were grown by vapor diffusion of pentane into a solution of 10 mg of [RuCl₂(=CH(o-OCH(Me)CO-*N-*Morpholine)Ph)(ImH₂Mes)] in 0.5 ml of dichloromethane at room temperature.

Fig. 2 shows a labeled view of the complex of formula F.

**Table X3**

| Crystal data and structure refinement for complex F | |
|---|---|
| Empirical formula | C35 H45 Cl2 N3 02 Ru |
| Formula weight | 777.67 |
| Crystal habit | Green, cubical |
| Temperature | 100 K |
| Wavelength | 0.7107 A |
| Crystal system, space group | Rhombohedral, R-3 |
| Unit cell dimensions | a = 28.620(4) A alpha = 90 deg. |
| | b = 28.620(4) A beta = 90 deg. |
| | c = 23.355(5) A gamma = 120 deg_{.} |
| Volume | 16567(5) A³ |
| Z, Calculated density | 18, 1.403 Mg/m³ |
| Absorption coefficient | 0.612 mm⁻¹ |
| F(000) | 7164 |
| Crystal size | 0.1 x 0.1 x 0.1 mm |
| Theta range for data collection | 2.98 to 26.34 deg. |
| Limiting indices | -34<=h<=26, -23<=k<=35, -20<=1<=29 |
| Reflections collected / unique | 18933 / 7461 [R(int) = 0.0426] |
| Completeness to theta = 26.37 | 99.5 % |
| Absorption correction | Semi-empirical from equivalents |
| Max. and min. transmission | 1.00000 and 0.87423 |
| Refinement method | Full-matrix least-squares on F² |
| Data / restraints / parameters | 7461 / 0 / 424 |
| Goodness-of-fit on F² | 1.076 |
| Final R indices [I>2sigma(I)] | R1 = 0.0662, wR2 = 0.1875 |
| R indices (all data) | R1 = 0.1032, wR2 = 0.2081 |
| Largest diff. peak and hole | 2.722 and -0.626 e.A⁻³ |

**Table X4**

| Selected Bond Lengths (A) and Angles (deg) for Complex F | |
|---|---|
| Bond Lengths (Å) | |
| Ru(1) C(10) | 1.833(6) |
| Ru(1) C(24) | 2.000(6) |
| Ru(1) O(4) | 2.221 (4) |
| Ru(1) O(5) | 2.4966 (43) |
| Ru(1) Cl(3) | 2.3566(15) |
| Ru(1) Cl(2) | 2.3787(16) |
| | |

| Bond Angles (deg) | |
|---|---|
| C(10)-Ru(1)-C(24) | 100.5(2) |
| C(10)-Ru(1)-O(4) | 79.6(2) |
| C(24)-Ru(1)-O(4) | 178.9(2) |
| C(10)-Ru(1)-O(5) | 145.73 (20) |
| C(24)-Ru(1)-O(5) | 113.76 (19) |
| O(4)-Ru(1)-O(5) | 66.08 (14) |
| C(10)-Ru(1)-Cl(2) | 94.76(18) |
| C(24)-Ru(1)-Cl(2) | 90.84(16) |
| O(4)-Ru(1)-Cl(2) | 88.04(11) |
| O(4)-Ru(1)-Cl(3) | 87.93(11) |
| Cl(2)-Ru(1)-Cl(3) | 167.82 (6) |

### Example 17

### Catalyst E, [RuCl₂(=CH(o-CH(Me)CONH₂)Ph)(ImH₂Mes)]

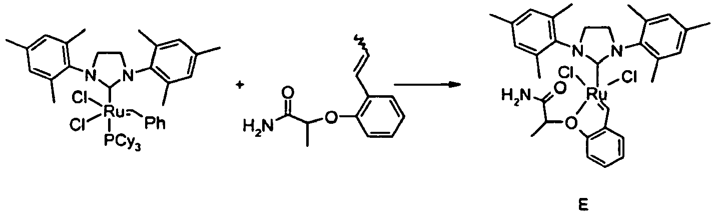

A suspension of 1.00 g (1.19 mmol) of [RuCl₂(PCy₃)(ImH₂Mes)(phenylmethylene)] (commercially available from Sigma-Aldrich Inc., St. Louis, USA), 0.15 g (1.47 mmol) of copper chloride and 0.30 g (1.47 mmol) of 2-[((*E,Z*)-2-propenyl)-phenoxy]-propionamide as a 3.5:1 mixture of *E*/*Z*-isomers in 75 ml of dichloromethane was stirred for 30 min at 40°C. The reaction mixture was evaporated to dryness at 40°C/10 mbar. The crude title product was dissolved in 100 ml of ethyl acetate and the formed suspension filtered. The filtrate was evaporated to dryness at 40 °C/10 mbar. The crude title product was purified by repeated digestion with pentane / THF to yield 0.41 g (53%) of the title compound as a green solid.

MS: 655.1 (M⁺). Anal. calcd. for C₃₁H₃₇Cl₂N₃O₂Ru: C, 56.79; H, 5.69; N, 6.41; Cl, 10.81. Found: C, 56.23; H, 5.59; N, 6.16; Cl, 10.84.

Crystals of the title compound suitable for X-ray crystal structure analysis were grown by vapor diffusion of pentane into a solution of 10 mg of [RuCl₂(=CH(o-OCH(Me)CONH₂) Ph)(ImH₂Mes)] in 0.5 ml of tetrahydrofuran at room temperature.

Fig. 3 shows a labeled view of the complex of formula E.

**Table X5**

| Crystal data and structure refinement for complex E | |
|---|---|
| Empirical formula | C33 H37 Cl2 N3 02.50 Ru |
| Formula weight | 687.63 |
| Crystal habit | Green, plates |
| Temperature | 100 K |
| Wavelength | 0.7107 A |
| Crystal system, space group | Monoclinic, C2/c |
| Unit cell dimensions | a = 31.8797(11) A alpha = 90 deg.. |
| | b = 15.8204(4) A beta = 110.956(3)deg.. |
| | c = 16.6158(5) A gamma = 90 deg |
| Volume | 7825.9(4) A³ |
| Z, Calculated density | 8, 1.167 Mg/m³ |
| Absorption coefficient | 0.566 mm⁻¹ |
| F(000) | 2832 |
| Crystal size | 0.2 x 0.1 x 0.05 mm |
| Theta range for data collection | 2.98 to 26.02 deg. |
| Limiting indices | -39<=h<=39, -19<=k<=19, -20<=1<=16 |
| Reflections collected / unique | 22018 / 7675 [R(int) = 0.1382] |
| Completeness to theta = 26.37 | 99.5 % |
| Absorption correction | None |
| Refinement method | Full-matrix least-squares on F² |
| Data / restraints / parameters | 7675 / 0 / 369 |
| Goodness-of-fit on F² | 1.103 |
| Final R indices [I>2sigma(I)] | R1 = 0.0650, wR2 = 0.1988 |
| R indices (all data) | R1 = 0.1050, wR2 = 0.2223 |
| Largest diff. peak and hole | 1.946 and -0.879 e.A⁻³ |

**Table X6**

| Selected Bond Lengths (Å) and Angles (deg) for Complex E | |
|---|---|
| Bond Lengths (A) | |
| Ru(1) C(18) | 1.839(5) |
| Ru(1) C(19) | 1.970(6) |
| Ru(1) O(4) | 2.257(4) |
| Ru(1) Cl(2) | 2.3371(15) |
| Ru(1) Cl(3) | 2.3415(15) |
| | |

| Bond Angles (deg) | |
|---|---|
| C(18)-Ru(1)-C(19) | 103.1(3) |
| C(18)-Ru(1)-O(4) | 79.6(2) |
| C(19)-Ru(1)-O(4) | 177.19(18) |
| C(18)-Ru(1)-Cl(2) | 96.61(19) |
| C(19)-Ru(1)-Cl(2) | 93.01(17) |
| O(4)-Ru(1)-Cl(2) | 87.38(11) |
| O(4)-Ru(1)-Cl(3) | 85.21(11) |
| Cl(2)-Ru(1)-Cl(3) | 161.50(6) |

### Example 18

### Catalyst No. G, [RuCl₂(=CH(o-OCH(Me)CONHPh)Ph)(ImH₂Mes)]

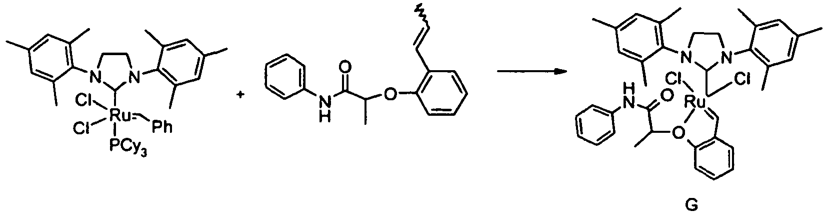

A suspension of 1.00 g (1.18 mmol) of [RuCl₂(PCy₃)(ImH₂Mes)(phenylmethylene)] (commercially available from Sigma-Aldrich Inc., St. Louis, USA), 0.13 g (1.30 mmol) of copper chloride and 0.38 g (1.30 mmol) of *N*-phenyl-2-[((*E,Z*)-2-propenyl)-phenoxy]-propionamide as a 4:1 mixture of *E*/*Z*-isomers in 75 ml of dichloromethane was stirred for 30 min at 40°C. The reaction mixture was evaporated to dryness at 40°C/10 mbar. The residue was stirred in 75 ml of ethyl acetate for 30 min at room temperature. The dark green suspension was filtered and the filtrate was evaporated to dryness at 40°C/10 mbar. The crude title product was purified by silica gel chromatography (cyclohexane/ethyl acetate 4:1) to yield 0.75 g (88% yield) of the title compound as a green powder.

MS: 731.1 (M⁺). Anal. calcd. for C₃₇H₄₁Cl₂N₃O₂Ru · ¹/₃ C₆H₁₂: C, 61.65; H, 5.97; N, 5.53; Cl, 9.33. Found: C, 61.83; H, 6.71; N, 5.35; Cl, 8.93.

### Example 19a

### Catalyst No. J, [RuCl₂(=CH(o-OCH(Me)CO-N-Pyrrolidine)Ph)(ImH₂Mes)]

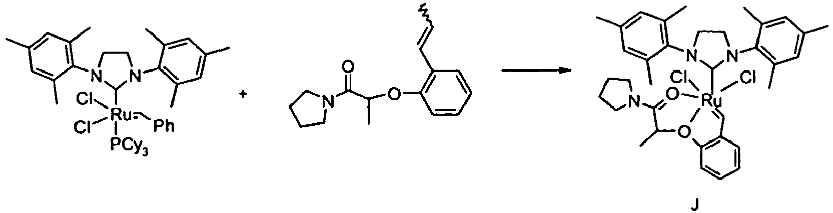

A suspension of 1.00 g (1.18 mmol) of [RuCl₂(PCy₃)(ImH₂Mes)(phenylmethylene)] (commercially available from Sigma-Aldrich Inc., St. Louis, USA), 0.13 g (1.30 mmol) of copper chloride and 0.36 g (1.30 mmol) of 2-[((*E,Z*)-2-propenyl)-phenoxy]-1-pyrrolidine-1-yl-propan-1-one as a 4:1 mixture of *E*/*Z*-isomers in 75 ml of dichloromethane was stirred for 30 min at 40°C. The reaction mixture was evaporated to dryness at 40°C/10 mbar. The residue was stirred in 60 ml of ethyl acetate for 30 min at room temperature. The dark green suspension was filtered and the filtrate was evaporated to dryness at 40°C/10 mbar. The crude title product was purified by silica gel chromatography (dichloromethane/methanol 98:2) to yield 0.52 g (62% yield) of the title compound as a green powder.

MS: 709.2 (M⁺). Anal. calcd. for C₃₅H₄₃Cl₂N₃O₂Ru · 0.85 CH₂Cl₂: C, 55.04; H, 5.76; N, 5.37, Cl 16.83. Found: C, 54.52; H, 5.74; N, 5.29, Cl 16.82.

### Example 19b

### Catalyst No. J, [RuCl₂(=CH(o-OCH(Me)CO-N-Pyrrolidine)Ph)(ImH₂Mes)]

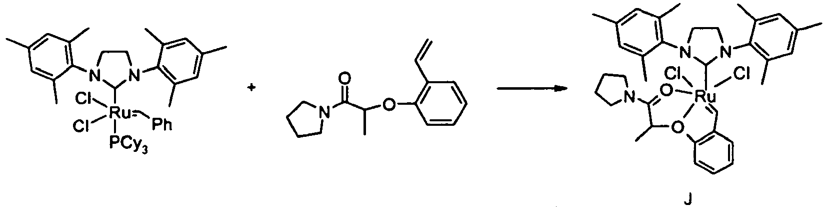

A solution of 2.00 g (2.11 mmol) of [RuCl₂(PCy₃)(ImH₂Mes)(3-phenyl-indenylidene)] (commercially available from Umicore AG, D-63457 Hanau-Wolfgang), 0.52 g (2.11 mmol) of 1-Pyrrolidin-1-yl-2-(2-vinylphenoxy)-propan-1-one in 60 ml of toluene was stirred for 6 h at 60°C. The reaction mixture was evaporated to dryness and the residual crude product was digested in 80 ml of ethyl acetate / pentane 3:5 for 30 min at room temperature, filtered and washed with 50 ml of ethyl acetate / pentane 1:4. The green crude product was digested in 10 ml of toluene for 30 min at 0-5-C, filtered and washed with 5 ml of toluene at 0-5°C and successively with 30 ml of hexane at room temperature to yield 0.52 g (34% yield) of the title compound as a green powder.

MS: 709.2 (M⁺). Anal. calcd. for C₃₅H₄₃Cl₂N₃O₂Ru: C, 59.23; H, 6.11; N, 5.92, Cl 9.99. Found: C, 58.92; H, 5.98; N, 5.36, Cl 9.14.

### Example 19c

### Catalyst No. K, [RuCl₂(=CH(o-OCH(Me)CO-N-Pyrrolidine)Ph)(PCy₃)]

### Step 1: [RuCl₂(=CH(o-OCH(Me)CO-N-Pyrrolidine)Ph)(PCy₃)]

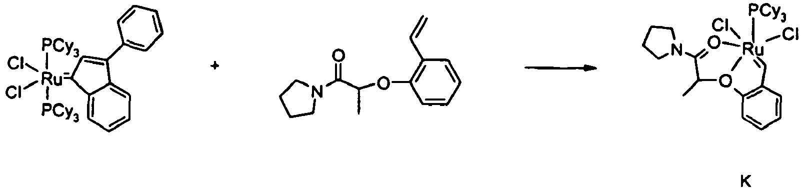

A solution of 20.00 g (21.70 mmol) of [RuCh(PCy₃)₂(3-phenyl-indenylidene)] (commercially available from Umicore AG, D-63457 Hanau-Wolfgang) and 5.32 g (21.70 mmol) of 1-Pyrrolidin-1-yl-2-(2-vinylphenoxy)-propan-l-one in 200 ml of toluene was stirred for 16 h at 100°C. The dark green solution was concentrated to a total volume of 100 ml, 200 ml of pentane were added and the formed dark green suspension was stirred for 30 min in an ice-bath. After filtration, the residual curde product K was washed with 110 ml of pentane / toluene 11:1 and successively with 100 ml of pentane and dried under vacuum at room temperature to yield 8.93 g (60%) of the title compound as a green powder.

MS: 683.2 (M⁺). ³1P-NMR (CDCl₃): δ 56.6 ppm.

### Example 19d

### Catalyst No. J, [RuCl₂(=CH(o-OCH(Me)CO-N-Pyrrolidine)Ph)(ImH₂Mes)]

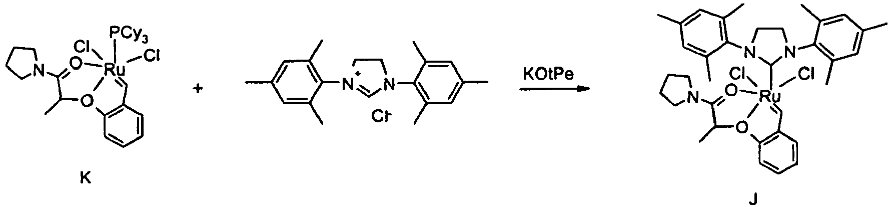

A suspension of 6.52 g (17.55 mmol) of 1,3-bis(2,4,6-trimethylphenyl)-imidazolidinium chloride (commercially available from Umicore AG, D-63457 Hanau-Wolfgang) and 11.40 ml (19.38 mmol) of potassium *tert*-.pentylate (1.7 M in toluene) in 400 ml of hexane was stirred for 10 min at 50°C. After the reaction mixture was evaporated to dryness, the white residue was redissolved in 400 ml of hexane and the formed suspension was transferred to a suspension of 4.00 g (5.85 mmol) of [RuCl₂(=CH(o-OCH(Me)CO-*N*-Pyrrolidine)Ph)(PCy₃)] in 400 ml of hexane. The resulting reaction mixture was stirred for 23 h at 50°C. The formed green suspension was filtered, the filter cake was washed with 60 ml of hexane and dissolved in 50 ml of dichloromethane. 140 ml of water was added, the organic layer was separated, dried over Na₂SO₄ and evaporated to dryness. The crude product was washed twice with 70 ml of ethyl acetate / pentane 1:5 and successively with 50 ml of pentane and dried under vacuum at room temperature to yield 2.75 g (66% yield) of the title compound as a green powder.

MS: 709.2 (M⁺). Anal. calcd. for C₃₅H₄₃Cl₂N₃O₂Ru · 0.9 CH₂Cl₂: C, 54.80; H, 5.74; N, 5.34, Cl 17.10. Found: C, 54.77; H, 5.76; N, 5.30, Cl 16.30.

### Example 20a

### Catalyst No. L, [RuCl₂(=CH(o-OCH(Me)CO-N-Pyrrolidine)Ph)(SIPrNap)]

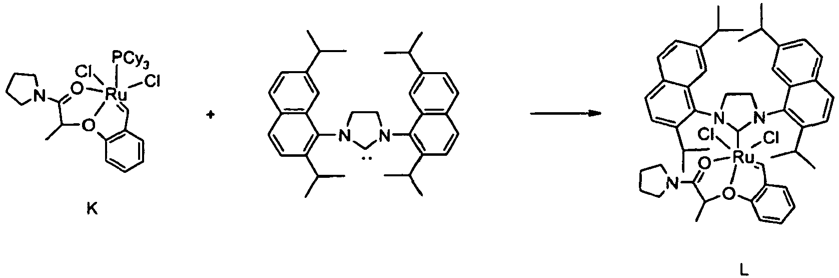

A suspension of 0.50 g (0.73 mmol) of [RuCl₂(=CH(o-OCH(Me)CO-*N*-Pyrrolidine)Ph)(PCy₃)] and 0.54 g (1.09 mmol) of SIPrNap (prepared according to R. Dorta et al., JACS 2008, 130, 6848) in 100 ml of hexane was stirred for 23 h at 50°C. The dark green suspension was filtered, the residue dissolved in 20 ml of dichloromethane and filtered. After evaporation off the solvent, the crude product was washed twice with 12 ml of ethyl acetate / pentane 1:2 and 10 ml of pentane and dried under vacuum at room temperature to yield 0.45 g (69%) of the title compound as a green powder.

MS: 893.3 (M⁺). Anal. calcd. for C₄₉H₅₉Cl₂N₃O₂Ru : C, 65.83; H, 6.65; N, 4.70, Cl 7.93. Found: C, 66.04; H, 6.74; N, 4.50, Cl 7.75.

### Example 20b

### Catalyst No. M, [RuCl₂(=CH(o-OCMe₂CO-N-Pyrrolidine)Ph)(ImH₂Mes)]

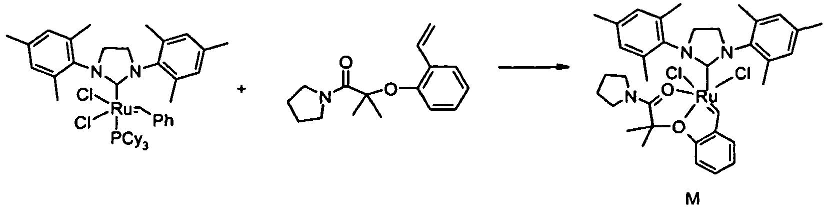

A suspension of 1.00 g (1.18 mmol) of [RuCl₂(PCy₃)(ImH₂Mes)(phenylmethylene)] (commercially available from Sigma-Aldrich Inc., St. Louis, USA), 0.14 g (1.41 mmol) of copper chloride and 0.37 g (1.41 mmol) of 2-methyl-1-pyrrolidin-1-yl-2-(2-vinyl-phenoxy)-propan-1-one in 80 ml of dichloromethane was stirred for 90 min at 40°C. The reaction mixture was evaporated to dryness at 40°C/10 mbar. The residue was stirred in 25 ml of ethyl acetate for 15 min at room temperature. The dark green suspension was filtered and the filtrate concentrated to a total volume of 5 ml. To the green solution, 15 ml of pentane were added and the formed suspension was stirred for 30 min at room temperature and filtered. The filter cake was washed with 40 ml of pentane and dried at 25°C/10 mbar over night to afford 0.68 g (80% yield) of the title compound as a green powder.

MS: 723.2 (M⁺).

### Example 20c

### Catalyst No. N, [RuCh(=CH(o-OCH₂CO-N-Pyrrolidine)Ph)(ImH₂Mes)]

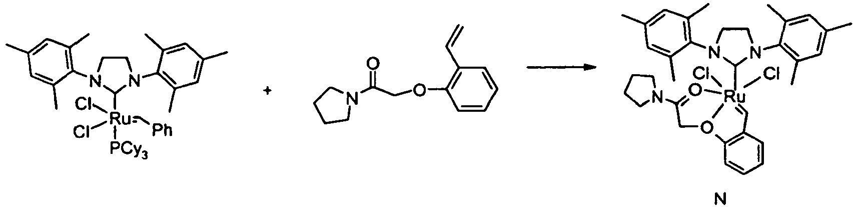

A solution of 1.00 g (1.18 mmol) of [RuCl₂(PCy₃)(ImH₂Mes)(phenylmethylene)] (commercially available from Sigma-Aldrich Inc., St. Louis, USA) and 0.28 g (1.20 mmol) of 1-pyrrolidin-1-yl-2-(2-vinyl-phenoxy)-ethanone in 30 ml of toluene was stirred for 6 h at 60°C. The formed green suspension was allowed to cool to room temperature. 50 ml of pentane was added to complete precipitation of the product. The suspension was stirred for 30 min at room temperature and filtered. The filter cake was washed with 100 ml of pentane and dried at 25°C/10 mbar over night to afford 0.59 g (72% yield) of the title compound as a green solid.

MS: 695.2 (M⁺). Anal. calcd. for C₃₄H₄₁Cl₂N₃O₂Ru: C, 58.70; H, 5.94; N, 6.04, Cl 10.19. Found: C, 58.56; H, 5.82; N, 5.90, Cl 10.14.

### Example 21

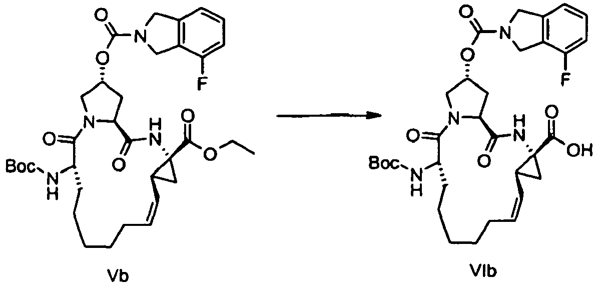

Preparation of (2R,6S,12Z,13aS,14aR,16aS)-6-[[(tert-butoxy)carbonyl]amino]-2-[[(4-fluoro-1,3-dihydro-2H-isoindol-2-yl)carbonyl]oxy]-1,2,3,6,7,8,9,10,11,13a,14,15,16,16a-hexadecahydro-5,16-dioxo]-cyclopropa[e]pyrrolo[1,2-a][1,4]diazacyclopentadecine-14a(5H)-carboxylic acid.

To a solution of 59.7 g (90.9 mmol) of RCM-ester Vb in 350 g of ethanol was added within one hour at 7°C 231 g of a sodium hydroxide solution (20% in water) and the resulting mixture was stirred for 6 hours at 5-10°C. The mixture was then treated at 10°C with 110 g of concentrated hydrochloric acid (37%). From the resulting mixture (approx. 800 ml) ethanol/water was distilled off until a residual volume of 350-400 ml was obtained in the reactor. The residue was treated at 40°C with 320 g of dichloromethane and 55 g of water and the resulting biphasic mixture was stirred at 40°C for 20 minutes. Stirring was stopped and the layers were allowed to separate for 15 minutes. The lower organic layer was separated. The aqueous layer was extracted with 64 g of dichloromethane and the combined organic layers were washed with water (1×55 g). From the organic layer dichloromethane was distilled off at atmospheric pressure and the removed solvent was continuously replaced by tetrahydrofuran; whereby the product crystallized out. In total, 600 g of tetrahydrofuran have been added. At the end of the distillation a volume of approx. 700 ml was adjusted in the reactor. After the distillation the suspension was heated to reflux for 5 hours. The suspension was then cooled to 0°C within 2 hours and stirred at this temperature for additional 3 hours. The crystals were filtered off, washed with 95 g of tetrahydrofuran and dried at 50°C/<30 mbar for 10 hours to afford 55.20 g (87% corrected yield) of the title compound as white crystals with a purity of 98.4 %(area), an assay of 90.2%(m/m) and a THF content of 8.5 %.

*MS: 627.3 (M⁺ - H).*

*¹H-NMR (400 MHz, DMSO-d₆): 12.2 (s, 1H), 8.73-8.66 (m, 1H), 7.39-7.31 (m, 1H), 7.22-7.02 (m, 3H), 5.57-5.46 (m, 1H), 5.31-5.21 (m, 2H), 4.67 (s, br, 4H), 4.47-4.38 (m, 1H), 4.29-4.20 (m, 1H), 3.98-3.88 (m, 1H), 3.71-3.62 (m, 1H), 2.70-2.55 (m, 1H), 2.29-2.08 (m, 3H), 1.75-1.0 (m, 11H), 1.10 and 1.07 (2s, 9H).*

### Example 22

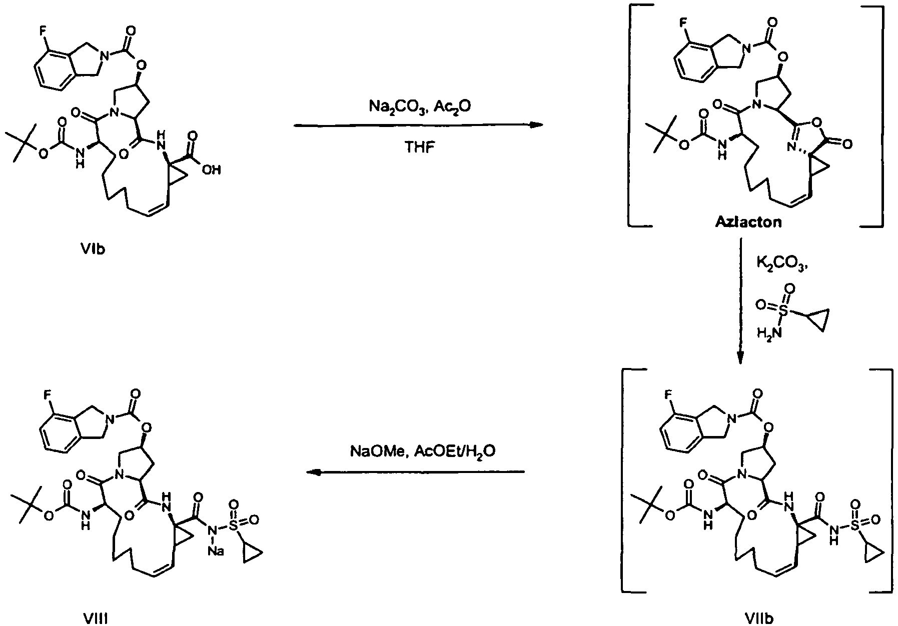

Preparation of Sodium ((2R,6S,13aS,14aR, 16aS,Z)-6-(tert-butoxycarbonylamino)-2-(4-fluoroisoiindoline-2-carbonyloxy)-5,16-dioxo-1,2,3,5,6,7,8,9,10,11,13a,15,16a-hexadecahydrocyclopropa[e]pyrrolo[1,2-a][1,4]diazacyclopentadecine-14a-carbonyl) (cyclopropylsulfonyl)amide (HCV protease inhibitor; compound VIII).

To a suspension of 30.0 g (0.043 mol) of carboxylic acid (product of example 20 with an assay of 90.2%(m/m)) and 14.0 g of sodium carbonate in 225 g of tetrahydrofuran was added at 45°C within 30 minutes 7.60 g (0.074 mol) of acetic acid anhydride and the resulting mixture was stirred at 45°C for 8 hours. To the resulting suspension was then added 30.2 g (0.17ml) of potassium carbonate and 8.0 g (0.065 mol) of cyclopropyl sulfonamide. The mixture was heated to 62°C and stirred at this temperature for 17 hours. The mixture was concentrated to a residual volume of 200 ml and then treated with 200 g of water. The biphasic mixture was stirred for 15 minutes and the layers were then allowed to separate. The lower aqueous phase was removed. The organic phase was diluted with 90 g of ethyl acetate and washed with 3% sulfuric acid (1×140 g) and water (3×130 g). The organic layer was concentrated to dryness and then diluted with 400 ml of ethyl acetate. Residual amounts of water were removed by a continuous azeotropic distillation with ethyl acetate. The mixture was then treated at 10°C with 20 ml of methanol, followed by 10.0 g of sodium methylate (30% in methanol). From the resulting mixture approx. 300 ml of ethyl acetate/methanol were then distilled off. The mixture was then treated at 34°C within one hour with 300 ml of ethyl acetate and 5 g of water. The resulting mixture was allowed to cool to ambient temperature within 4 hours. The crystals were filtered off, washed with 80 ml of ethyl acetate and dried at 80°C/<30 mbar for 20 hours to afford 30.4 g (87% corrected yield) of the title compound as white crystals with an assay of 92.7 %(m/m).

*MS: 732.28 (M⁺ + H), 676.23, 632.25.*

*¹H-NMR (400 MHz, DMSO-d₄): 7.89-7.80 (m, 1H), 7.39-7.31 (m, 1H), 7.21-7. 06 (m, 2H), 6.97-6.90 (m, 1H), 5.49-4.41 (m, 1H), 5.31-5.21 (m, 2H), 4.66 (s, br, 4H), 4.45-4.35 (m, 1H), 4.19-4.08(m, 2H), 3.91-3.81 (m, 1H), 2.68-2.58(m, 1H), 2.30-2.14 (m, 3H), 2.0-1.2 (m, 12H), 1.17 and 1.14 (2s, 9H), 0.78-0.69 (m, 2H), 0.62-0.53 (m, 2H).*

## Claims

1. A compound of the formula 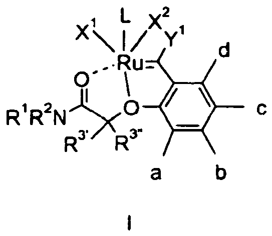 wherein the dotted line either signifies the existence of a bond or no bond;
L is a neutral ligand selected from 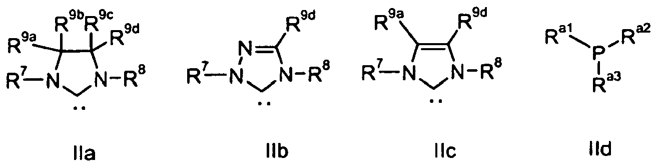 wherein R⁷ and R⁸ independently of each other are C₁₋₆-alkyl, aryl, C₂₋₆- alkenyl or 1-adamantyl and
R^{9a-d} are independently of each other hydrogen, C₁₋₆-alkyl, C₂₋₆- alkenyl or aryl, or R^{9b} and R^{9c} or R^{9a} and R^{9d} taken together form a-(CH₂)₄-bridge;
or in formula IIc R^{9a} and R^{9d} are both halogen;
R^{a1}, R^{a2} and R^{a3} independently of each other are C₁₋₆-alkyl, C₃₋₈-cycloalkyl, aryl, heteroaryl or R^{a1} and R^{a2} or R^{a2} and R^{a3} or R^{a1} and R^{a3} taken together form a 1,5-bridged cyclooctyl group;
X¹ and X² independently of each other are anionic ligands;
Y¹ is hydrogen, C₁₋₆-alkyl, C₃₋₈-cycloalkyl, C₂₋₆-alkenyl, C₂₋₆-alkynyl, C₁₋₆-alkoxy, C₂₋₆-alkenyloxy, C₂₋₆-alkynyloxy, aryloxy, C₁₋₆-alkoxycarbonyl, C₁₋₆-alkylthio, aryl, arylthio, C₁₋₆-alkylsulfonyl, C₁₋₆-alkylsulfinyl;
a, b, c and d independently of each other have the meaning of hydrogen, C₁₋₆-alkyl, halogen-C₁₋₆-alkyl, C₂₋₆-alkenyl, C₂₋₆-alkynyl, C₁₋₆-alkoxy, C₂₋₆-alkenyloxy, C₂₋₆-alkynyloxy, C₁₋₆-alkylcarbonyl, aryl, hydroxy, aryloxy, nitro, C₁₋₆-alkoxycarbonyl, amino, mono-C₁₋₆-alkyl-or di-C₁₋₆-alkylamino, halogen, thio, C₁₋₆-alkylthio, arylthio, C₁₋₆-alkylsulfonyl, C₁₋₆-alkylsulfinyl, arylsulfonyl, SO₃H, C₁₋₆-alkylcarbonyl amino, aryl carbonyl amino, C₁₋₆-alkyl sulfonyl amino, aryl sulfonyl amino, halogen-C₁₋₆-alkyl sulfonyl amino, SO₃-C₁₋₆-alkyl or OSi(C₁₋₆-alkyl)₃ and SO₂-NR'R" wherein R' and R" independently of each other have the meaning of hydrogen, aryl or C₁₋₆-alkyl or R' and R" together with the N atom form a cycle;
R¹ and R² independently of each other are hydrogen, C₁₋₆-alkyl, C₃₋₈-cycloalkyl, aryl, aryl-C₁₋₆-alkyl or
R¹ and R² together with the N atom form a 5 to 8 member cycle which may contain nitrogen, oxygen or sulfur as additional hetero atom;
R^{3'} and R^{3"} independently of each other are hydrogen, C₁₋₆-alkyl, C₃₋₈-cycloalkyl, aryl, aryl-C₁₋₆-alkyl.

2. Compounds of claim 1 wherein the dotted line signifies a bond and generates a hexacoordinated Ru (II) complex.

3. A compound of claim 2 wherein L is 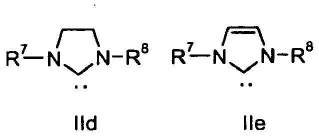 wherein R⁷ and R⁸ independently of each other are C₁₋₆-alkyl, 1-adamantyl, a phenyl group which is di- or tri-substituted with C₁₋₆-alkyl or naphthyl which is di- or tri-substituted with C₁₋₆-alkyl.

4. A compound of claim 3 wherein R⁷ and R⁸ is 2, 4, 6-trimethylphenyl or 2,7-diisopropylnaphthyl.

5. A compound of claim 1 or 2, wherein X¹ and X² independently of each other are halogen.

6. A compound of claim 5, wherein X¹ and X² are chloro.

7. A compound of claim 1 or 2, wherein Y¹ is hydrogen.

8. A compound of claim 1 or 2, wherein a, b and d is hydrogen.

9. A compound of claim 1 or 2, wherein c is hydrogen, halogen, nitro, C₁₋₆-alkylcarbonyl amino, aryl carbonyl amino, aryl sulfonyl amino, alkyl sulfonyl amino, halogen-C₁₋₆-alkyl sulfonyl amino, SO₂-NR'R" wherein R' and R" independently of each other have the meaning of hydrogen, C₁₋₆-alkyl, aryl or R' and R" together with the N atom form a cycle.

10. A compound of claims 1 or 2, wherein R¹ and R² independently of each other are hydrogen, C₁₋₆-alkyl or
R¹ and R² together with the N atom form a 6 member cycle which contains oxygen as additional hetero atom.

11. A compound of claims 1 or 2, wherein R^{3'} and R^{3"} independently of each other are hydrogen or C₁₋₆-alkyl.

12. Compounds of claims 1 to 11 selected from
| | |
|---|---|
| 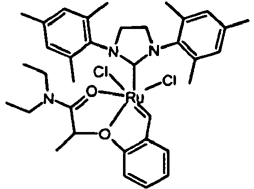 | [RuCl₂(=CH(o-OCH(Me)CONEt₂)Ph)(ImH₂Mes)] |
| 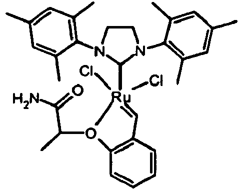 | [RuCl₂(=CH(o-OCH(Me)CONH₂)Ph)(ImH₂Mes)] |
| 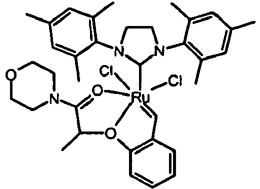 | [RuCl₂(=CH(o-OCH(Me)CO-N Morpholine)Ph)(ImH₂Mes)] |
| 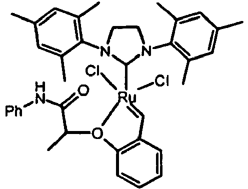 | (RuCl₂(=CH(o-OCH(Me)CONHPh)Ph)(ImH₂Mes)] |
| 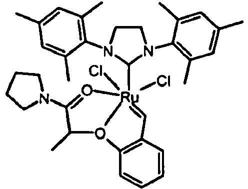 | [RuCl₂(=CH(o-OCH(Me)CO-N Pyrrolidine)Ph)(ImH₂Mes)] |
| 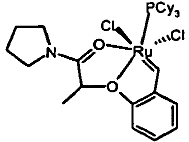 | [RuCl₂(=CH(o-OCH(Me)CO-*N*-Pyrrolidine)Ph)(PCy₃₎] |
| 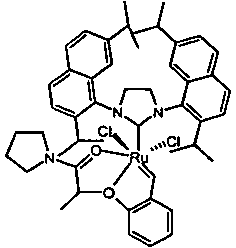 | [RuCl₂(=CH(o-OCH(Me)CO-*N*-Pyrrolidine)Ph)(SIPrNap)] |
| 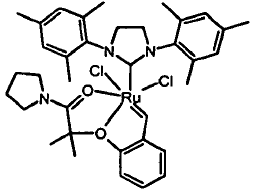 | [RuCl₂(=CH(o-OCMe₂CO-*N*-Pyrrolidine)Ph)(ImH₂Mes)] |
| 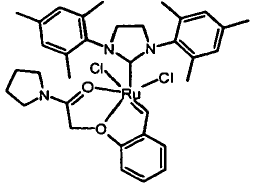 | [RuCl₂(=CH(o-OCH₂CO-*N*-Pyrrolidine)Ph)(ImH₂Mes)] |

13. Process for the preparation of compounds of formula I comprising the conversion of a preligand of the formula 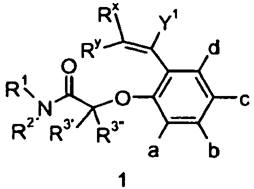 wherein R¹, R² and R^{3'} and R^{3"}, Y¹ and a,b,c,d are as defined above;
R^{x} and R^{y} independently of each denote hydrogen, C₁₋₆-alkyl optionally substituted by one or more halogen atoms or aryl optionally substituted by one or more halogen atoms or by C₁₋₆-alkyl
with a Ru-complex selected from 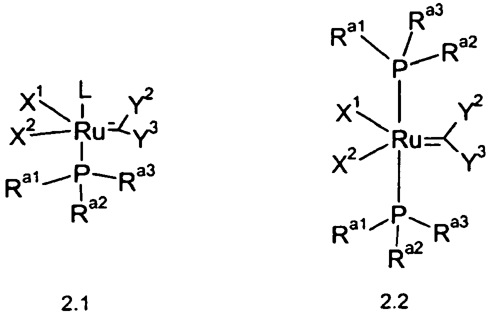 wherein L, X¹ and X² are as defined above;
Y² and Y³ independently of each other are hydrogen, C₁₋₆-alkyl, C₂₋₆-alkenyl, C₂₋₆-alkynyl, C₁₋₆-alkylthio, aryl, arylthio, C₁₋₆-alkylsulfonyl, C₁₋₆-alkylsulfinyl,
or Y² and Y³ taken together form a cycle of the type 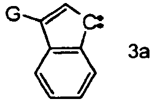 with G being hydrogen or aryl;
or
Y² and Y³ together form a cumulenyl group of type 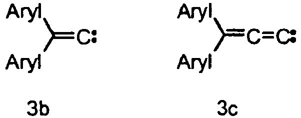 R^{a1}, R^{a2} and R^{a3} independently of each other are C₁₋₆alkyl, C₃₋₈-cycloalkyl, aryl, heteroaryl or R^{a1} and R^{a2} or R^{a2} and R^{a3} or R^{a1} and R^{a3} form together a 1,5-bridged cyclooctyl group.

14. Process of claim 13, **characterized in that** the conversion is performed in an inert solvent at a temperature of 0°C to 80°C.

15. Process of claims 13 and 14, **characterized in that** the conversion is performed in the presence of CuCl.

16. Preligands of formula 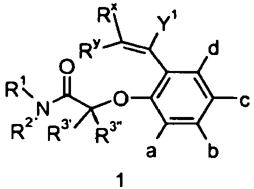 wherein R¹, R² and R^{3'}, R^{3"}, R^{x}, R^{y}, Y¹ and a, b, c, d are as defined above
selected from

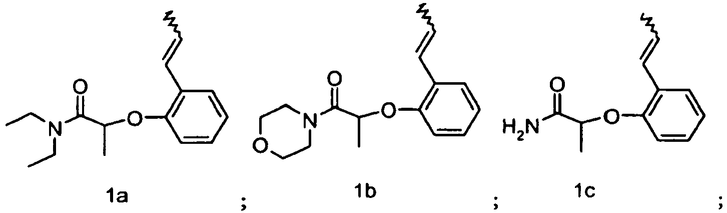

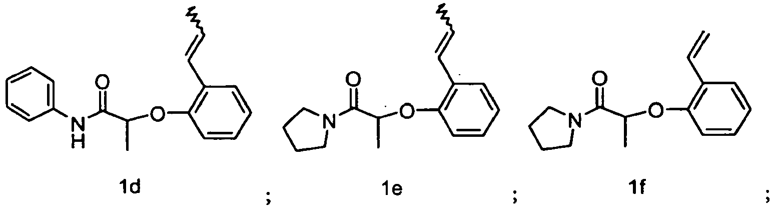

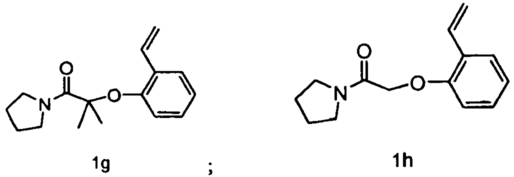

17. Use of the compounds of claims 1 to 12 in metathesis reactions.

18. Process for the manufacture of a macrocyclic compound of formula 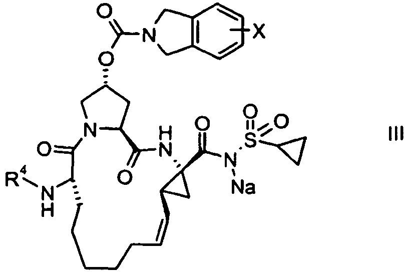 wherein R⁴ is an amino protecting group and X is a halogen atom, comprising
subjecting a diene compound of formula 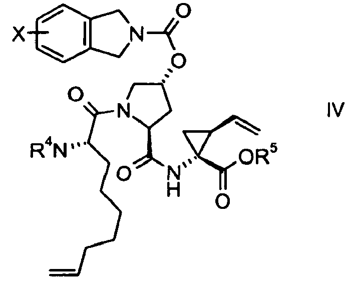 wherein R⁴ is an amino protecting group, R⁵ is C₁₋₄-alkyl and X is halogen to a ring closing metathesis reaction in the presence of a compound of claims 1 to 12.

19. Process for the manufacture of a macrocyclic compound of formula 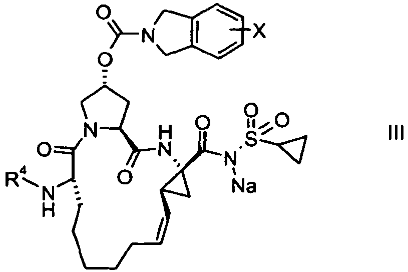 wherein R⁴ is an amino protecting group and X is a halogen atom, comprising the steps
a) subjecting a diene compound of formula 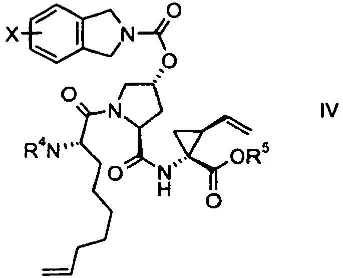 wherein R⁴ is an amino protecting group, R⁵ is C₁₋₄-alkyl and X is halogen to a ring closing metathesis reaction in the presence of a compound of claims 1 to 12
to form a macrocyclic ester of the formula 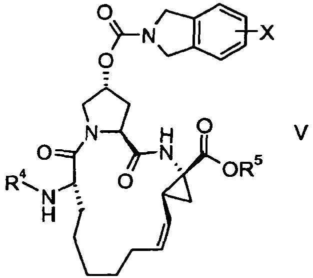 wherein R⁴ is an amino protecting group, R⁵ is C₁₋₄-alkyl and X is halogen;
b) hydrolyzing the macrocyclic ester of formula V in the presence of a base to form the
macrocyclic acid of the formula 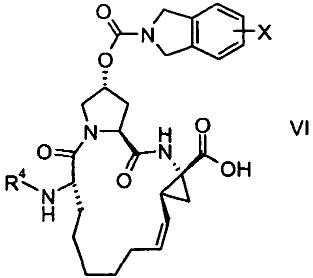 wherein R⁴ is an amino protecting group and X is halogen;
c) forming the macrocyclic sulfonamide of formula 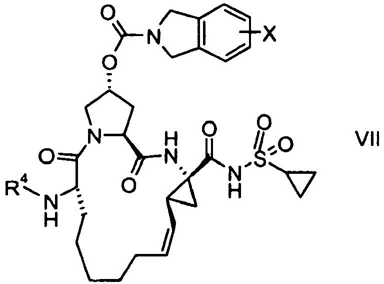 wherein R⁴ is an amino protecting group and X is halogen by coupling the macrocyclic acid of formula VI with cyclopropyl sulfonamide and
d) treating the macrocyclic sulfonamide of formula XXI with a sodium base to form the macrocyclic compound of formula III.

20. Process of claim 18 or 19, wherein the compounds of claims 1 to 12 are selected from the compounds:
| | |
|---|---|
| 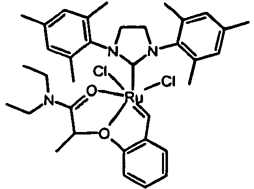 | [RuCl₂(=CH(o-OCH(Me)CONEt₂)Ph)(ImH₂Mes)] |
| 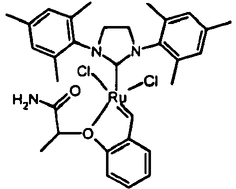 | [RuCl₂(=CH(o-OCH(Me)CONH₂)Ph)(ImH₂Mes)] |
| 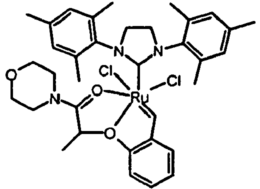 | [RuCl₂(=CH(o-OCH(Me)CO-N Morpholine)Ph)(ImH₂Mes)] |
| 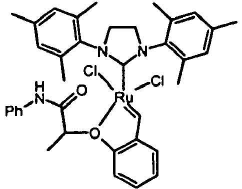 | [RuCl₂(=CH(o-OCH(Me)CONHPh)Ph)(ImH₂Mes)] |
| 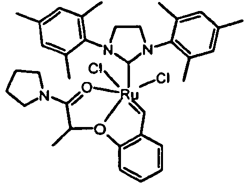 | [RuCl₂(=CH(o-OCH(Me)CO-N Pyrrolidine)Ph)(ImH₂Mes)] |
| 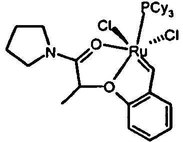 | [RuCl₂(=CH(o-OCH(Me)CO-N Pyrrolidine)Ph)(PCy₃)] |
| 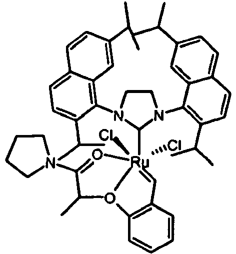 | [RuCl₂(=CH(o-OCH(Me)CO-*N*-Pyrrolidine)Ph)(SIPrNap)] |
| 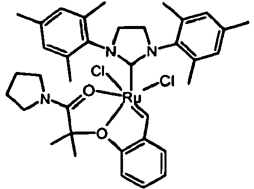 | [RuCl₂(=CH(o-OCMe₂CO-*N* Pyrrolidine)Ph)(ImH₂Mes)] |
| 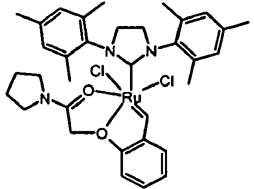 | [RuCl₂(=CH(o-OCH₂CO-*N*-Pyrrolidine)Ph)(ImH₂Mes)] |

21. Process of claims 18 to 20, wherein the ring closing metathesis reaction in step a) is performed in an organic solvent at 20°C to 140°C.

22. Process of claims 18 to 21, wherein the ring closing metathesis reaction in step a) is performed with a substrate to catalyst ratio in the range of 20 to 10000.

23. Process of claims 19 to 22, wherein the hydrolysis in step b) is performed with an aqueous alkali hydroxide solution at a temperature of 0°C to 40°C.

24. Process of claims 19 to 23, wherein the macrocyclic acid of formula VI obtained in step b) is isolated by way of extraction with dichloromethane and a subsequent crystallization in tetrahydrofuran.

25. Process of claims 19 to 24, wherein the formation of the macrocyclic sulfonamide of formula VII in step c) is in a first step the reaction of the macrocyclic acid of formula VI with acetic acid anhydride in the presence of an inorganic base and a suitable organic solvent into an azlacton intermediate of the formula 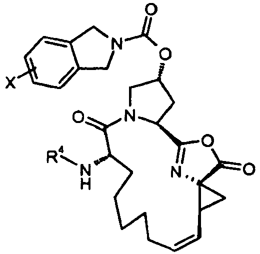 wherein R⁴ is an amino protecting group and X is halogen and the subsequent reaction of the azlacton with cyclopropyl sulfonamide in the presence of an inorganic base to the macrocyclic sulfonamide of formula VII.

26. Process of claim 19 to 25, wherein the sodium base used for the treatment of the macrocyclic sulfonamide of the formula VII in step d) is sodium hydroxide, sodium methylate or sodium ethoxide.

27. Process of claims 18 to 26, wherein
R⁴ is Boc;
R⁵ is ethyl;
and the moiety of the formula 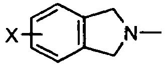 stands for

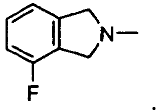

## Patentansprüche

1. Eine Verbindung der Formel 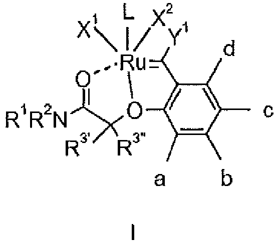 wobei die gestrichelte Linie entweder das Vorhandensein einer Bindung oder keiner Bindung anzeigt;
L ein neutraler Ligand ist, ausgewählt aus 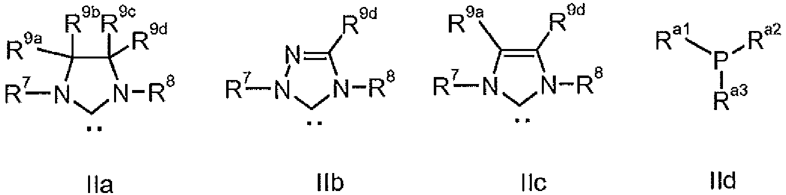 wobei R⁷ und R⁸ unabhängig voneinander C₁₋₆-Alkyl, Aryl, C₂₋₆-Alkenyl oder 1-Adamantyl sind und
R^{9a-d} unabhängig voneinander Wasserstoff, C₁₋₆-Alkyl, C₂₋₆-Alkenyl oder Aryl sind oder R^{9b} und R^{9c} oder R^{9a} und R^{9d} zusammen genommen eine -(CH₂)₄-Brücke bilden;
oder R^{9a} und R^{9d} in Formel IIc beide Halogen sind;
R^{a1}, R^{a2} und R^{a3} unabhängig voneinander C₁₋₆-Alkyl, C₃₋₈-Cycloalkyl, Aryl, Heteroaryl sind oder R^{a1} und R^{a2} oder R^{a2} und R^{a3} oder R^{a1} und R^{a3} zusammen genommen eine Cyclooctylgruppe mit einer 1,5-bücke bilden;
X¹ und X² unabhängig voneinander anionische Liganden sind;
Y¹ Wasserstoff, C₁₋₆-Alkyl, C₃₋₈-Cycloalkyl, C₂₋₆-Alkenyl, C₂₋₆-Alkinyl, C₁₋₆-Alkoxy, C₂₋₆-Alkenyloxy, C₂₋₆-Alkinyloxy, Aryloxy, C₁₋₆-Alkoxycarbonyl, C₁₋₆-Alkylthio, Aryl, Arylthio, C₁₋₆-Alkylsulfonyl, C₁₋₆-Alkylsulfinyl ist;
a, b, c und d unabhängig voneinander Wasserstoff, C₁₋₆-Alkyl, Halogen-C₁₋₆-alkyl, C₂₋₆-Alkenyl, C₂₋₆-Alkinyl, C₁₋₆-Alkoxy, C₂₋₆-Alkenyloxy, G₂₋₆-Alkinyloxy, C₁₋₆-Alkylcarbonyl, Aryl, Hydroxy, Aryloxy, Nitro, C₁₋₆-Alkoxycarbonyl, Amino, Mono-C₁₋₆-alkyl- oder Di-C₁₋₆-alkylamino, Halogen, Thio, C₁₋₆-Alkylthio, Arylthio, C₁₋₆-Alkylsulfonyl, C₁₋₆-Alkylsulfinyl, Arylsulfonyl, SO₃H, C₁₋₆-Alkylcarbonylamino, Arylcarbonylamino, C₁₋₆-Alkylsulfonylamino, Arylsulfonylamino, Halogen-C₁₋₆-alkylsulfonylamino, SO₃-C₁₋₆-Alkyl oder OSi(C₁₋₆-Alkyl)₃ und SO₂-NR'R" bedeuten, wobei R' und R" unabhängig voneinander Wasserstoff, Aryl oder C₁₋₆-Alkyl bedeuten oder R' und R" zusammen mit dem N-Atom einen Ring bilden;
R¹ und R² unabhängig voneinander Wasserstoff, C₁₋₆-Alkyl, C₃₋₈-Cycloalkyl, Aryl, Aryl-C₁₋₆-alkyl sind oder
R¹ und R² zusammen mit dem N-Atom einen Ring mit 5 bis 8 Gliedern bilden, der Stickstoff, Sauerstoff oder Schwefel als zusätzliches Heteroatom enthalten kann;
R^{3'} und R^{3"} unabhängig voneinander Wasserstoff, C₁₋₆-Alkyl, C₃₋₈-Cycloalkyl, Aryl, Aryl-C₁₋₆-alkyl sind.

2. Verbindungen nach Anspruch 1, wobei die gestrichelte Linie eine Bindung kennzeichnet und einen sechsfach koordinierten Ru(II)-Komplex erzeugt.

3. Eine Verbindung nach Anspruch 2, wobei L 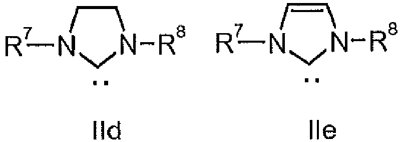 ist,
wobei R⁷ und R⁸ unabhängig voneinander C₁₋₆-Alkyl, 1-Adamantyl, eine Phenylgruppe, die zweifach oder dreifach mit C₁₋₆-Alkyl substituiert ist, oder Naphthyl, das zweifach oder dreifach mit C₁₋₆-Alkyl substituiert ist, sind.

4. Eine Verbindung nach Anspruch 3, wobei R⁷ und R⁸ gleich 2,4,6-Trimethylphenyl oder 2,7-Diisopropylnaphthyl sind.

5. Eine Verbindung nach Anspruch 1 oder 2, wobei X¹ und X² unabhängig voneinander Halogen sind.

6. Eine Verbindung nach Anspruch 5, wobei X¹ und X² Chlor sind.

7. Eine Verbindung nach Anspruch 1 oder 2, wobei Y¹ Wasserstoff ist.

8. Eine Verbindung nach Anspruch 1 oder 2, wobei a, b und d Wasserstoff sind,

9. Eine Verbindung nach Anspruch 1 oder 2, wobei c Wasserstoff, Halogen, Nitro, C₁₋₆-Alkylcarbonylamino, Arylcarbonylamino, Arylsulfonylamino, Alkylsulfonylamino, Halogen-C₁₋₆-alkylsulfonylamino, SO₂-NR'R" ist, wobei R' und R" unabhängig voneinander Wasserstoff, C₁₋₆-Alkyl, Aryl bedeuten oder R' und R" zusammen mit dem N-Atom einen Ring bilden.

10. Eine Verbindung nach Anspruch 1 oder 2, wobei R¹ und R² unabhängig voneinander Wasserstoff, C₁₋₆-Alkyl sind oder
R¹ und R² zusammen mit dem N-Atom einen Ring mit 6 Gliedern bilden, der Sauerstoff als zusätzliches Heteroatom enthält.

11. Eine Verbindung nach Anspruch 1 oder 2, wobei R³' und R³" unabhängig voneinander Wasserstoff oder C₁₋₆-Alkyl sind.

12. Verbindungen der Ansprüche 1 bis 11, ausgewählt aus
| | |
|---|---|
| 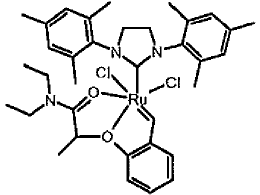 | [RuCl₂(=CH(o-OCH(Me)CONEt₂)Ph)(ImH₂Mes)] |
| 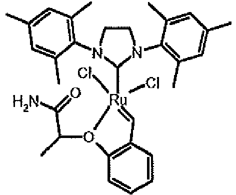 | [RuCl₂(=CH(o-OCH(Me)CONH₂)Ph)(ImH₂Mes)] |
| 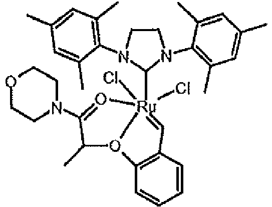 | [RuCl₂(=CH(o-OCH(Me)CO-*N*-Morpholin)Ph)(ImH₂Mes)] |
| 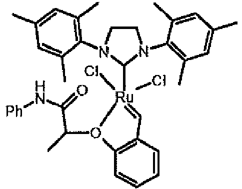 | [RuCl₂(=CH(o-OCH(Me)CONHPh)Ph)(ImH₂Mes)] |
| 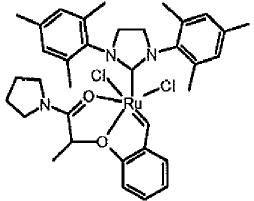 | [RuCl₂(=CH(o-OCH(Me)CO-*N*-Pyrrolidin)Ph)(ImH₂Mes)] |
| 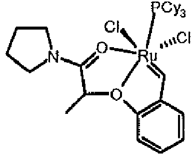 | [RuCl₂(=CH(o-OCH(Me)CO-*N*-Pyrrolidin)Ph)(PCy₃)] |
| 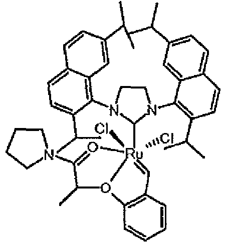 | [RuCl₂(=CH(o-OCH(MC)CO-*N*-Pyrrolidin)Ph)(SIPrNap)] |
| 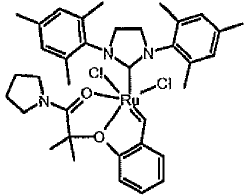 | FRuCl₂(=CH(o-OCMe₂CO-*N*-Pyrrolidin)Ph)(ImH₂Mes)] |
| 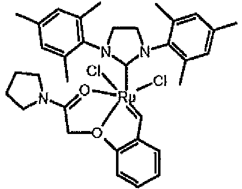 | [RuCl₂(=CH(o-OCH₂CO-*N*-Pyrrolidin)Ph)(ImH₂Mes)] |

13. Verfahren zur Herstellung von Verbindungen der Formel I, umfassend das Umwandeln einer Ligandenvorstufe der Formel 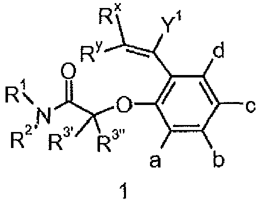 wobei R¹, R² und R^{3'} und R^{3"}, Y¹ und a, b, c, d wie vorstehend definiert sind;
R^{x} und R^{y} unabhängig voneinander Wasserstoff, C₁₋₆-Alkyl, gegebenenfalls substituiert mit einem oder mehreren Halogenatomen, oder Aryl, gegebenenfalls substituiert mit einem oder mehreren Halogenatomen oder mit C₁₋₆-Alkyl, bezeichnen
mit einem Ru-Komplex, ausgewählt aus 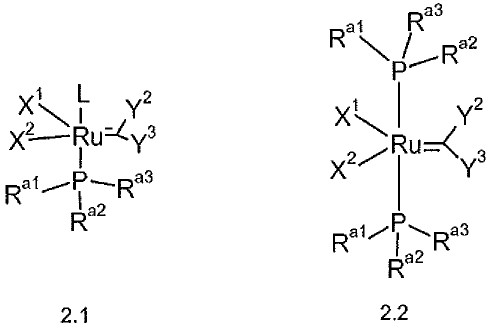 wobei L, X¹ und X² wie vorstehend definiert sind;
Y² und Y³ unabhängig voneinander Wasserstoff, C₁₋₆-Alkyl, C₂₋₆-Alkenyl, C₂₋₆-Alkinyl, C₁₋₆-Alkylthio, Aryl, Arylthio, C₁₋₆-Alkylsulfonyl, C₁₋₆-Alkylsulfinyl sind,
oder Y² und Y³ zusammen genommen einen Ring des Typs 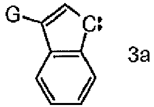 bilden,
wobei G Wasserstoff oder Aryl ist;
oder
Y² und Y³ zusammen einen Cumulenylrest des Typs 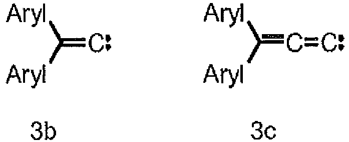 bilden
R^{a1}, R^{a2} und R^{a3} unabhängig voneinander C₁₋₆-Alkyl, C₃₋₈-Cycloalkyl, Aryl, Heteroaryl sind oder R^{a1} und R^{a2} oder R^{a2} und R^{a3} oder R^{a1} und R^{a3} zusammen eine Cyclooctylgruppe mit einer 1,5-Brücke bilden.

14. Verfahren nach Anspruch 13, **dadurch gekennzeichnet, dass** die Umwandlung in einem inerten Lösungsmittel bei einer Temperatur von 0°C bis 80°C durchgeführt wird.

15. Verfahren nach den Ansprüche 13 und 14, **dadurch gekennzeichnet, dass** die Umwandlung in Gegenwart von CuCl durchgeführt wird.

16. Ligandenvorstufen der Formel 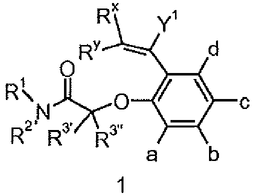 wobei R¹, R² und R³', R³", R^{x}, R^{y}, Y¹ und a, b, c, d wie vorstehend definiert sind, ausgewählt aus

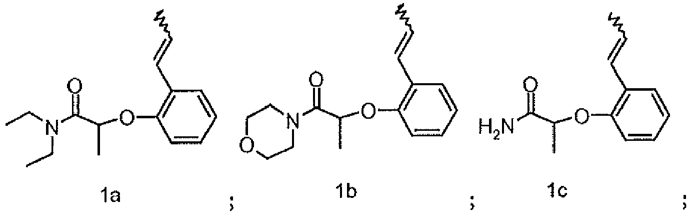

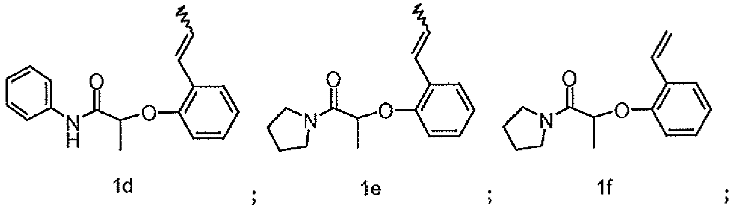

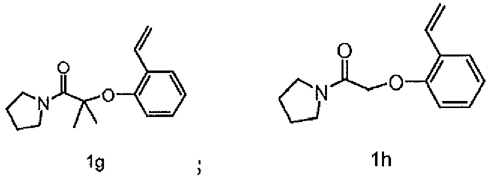

17. Verwendung der Verbindungen der Ansprüche 1 bis 12 bei Metathesereaktionen.

18. Verfahren zur Herstellung einer makrocyclischen Verbindung der Formel 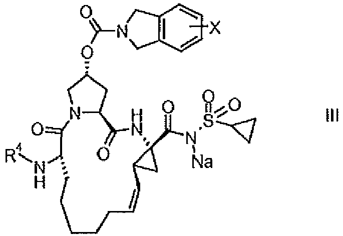 wobei R⁴ eine Amino-Schutzgruppe ist und X ein Halogenatom ist, umfassend Unterziehen einer Dienverbindung der Formel 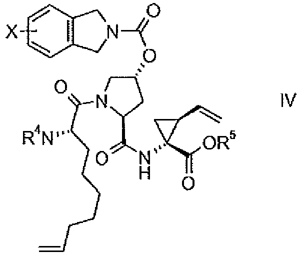 wobei R⁴ eine Amino-Schutzgruppe ist, R⁵ C₁₋₄-Alkyl ist und X Halogen ist, einer Ringschlusstnetathesereaktion in Gegenwart einer Verbindung der Ansprüche 1 bis 12.

19. Verfahren zur Herstellung einer makrocyclischen Verbindung der Formel 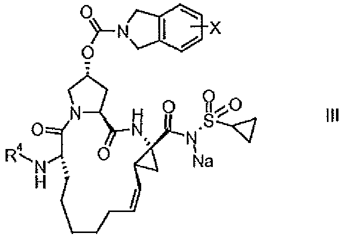 wobei R⁴ eine Amino-Schutzgruppe ist und X ein Halogenatom ist, umfassend die Schritte
a) Unterziehen einer Dienverbindung der Formel 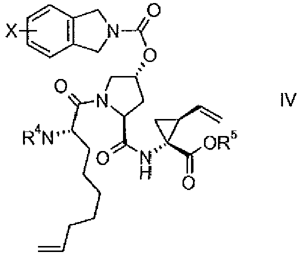 wobei R⁴ eine Amino-Schutzgruppe ist, R⁵ C₁₋₄-Alkyl ist und X Halogen ist, einer Ringschlussmetathesereaktion in Gegenwart einer Verbindung der Ansprüche 1 bis 12, um einen makrocyclischen Ester der Formel 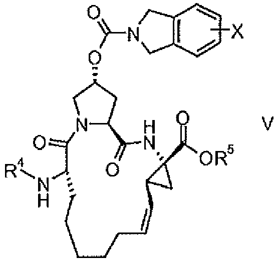 zu bilden,
wobei R⁴ eine Amino-Schutzgruppe ist, R⁵ C₁₋₄-Alkyl ist und X Halogen ist;
b) Hydrolysieren des makrocyclischen Esters der Formel V in Gegenwart einer Base, um die makrocyclische Säure der Formel 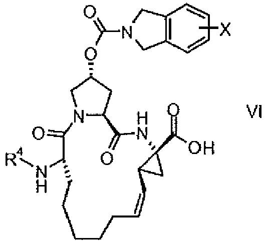 zu bilden,
wobei R⁴ eine Amino-Schutzgruppe ist und X Halogen ist;
c) Bilden des makrocyclischen Sulfonamids der Formel 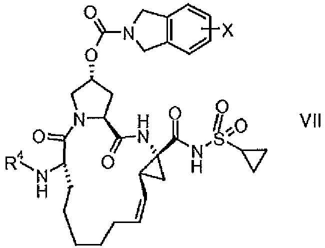 wobei R⁴ eine Amino-Schutzgruppe ist und X Halogen ist, durch Kuppeln der makrocyclischen Säure der Formel VI mit Cyclopropylsulfonamid und
d) Behandeln des makrocyclischen Sulfonamids der Formel VII mit einer Natriumbase, um die makrocyclische Verbindung der Formel III zu bilden.

20. Verfahren nach Anspruch 18 oder 19, wobei die Verbindungen der Ansprüche 1 bis 12 ausgewählt sind aus den Verbindungen:
| | |
|---|---|
| 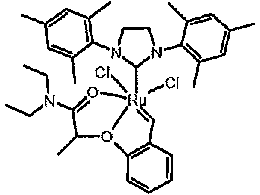 | [RuCl₂(=CH(o-OCH(Me)CONEt₂)Ph)(ImH₂Mes)] |
| 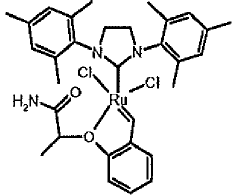 | [RuCl₂(=CH(o-OCH(Me)CONH₂)Ph)(ImH₂Mes)] |
| 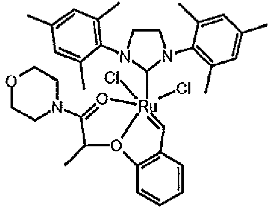 | [RuCl₂(=CH(o-OCH(Me)CO-*N*-Morpholin)Ph)(ImH₂Mes)] |
| 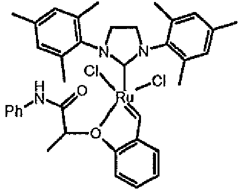 | [RuCl₂(=CH(o-OCH(Me)CONHPh)Ph)(ImH₂Mes)] |
| 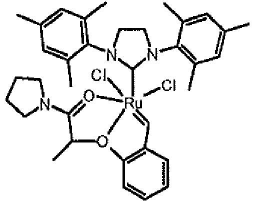 | [RuCl₂(-CH(o-OCH(Me)CO-*N*-Pyrrolidin)Ph)(ImH₂Mes)] |
| 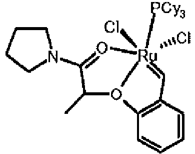 | [RuCl₂(=CH(o-OCH(Me)CO-*N*-Pyrrolidin)Ph)(PCy₃)] |
| 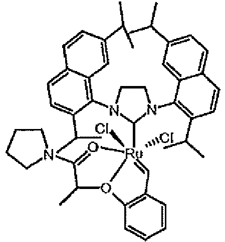 | [RuCl₂(=CH(o-OCH(Me)CO-*N*-Pyrrolidin)Ph)(SIPrNap)] |
| 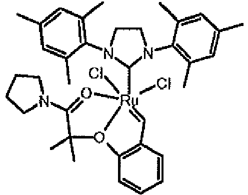 | [RuCl₂(=CH(o-OCMe₂CO-*N*-Pyrrolidin)Ph)(ImH₂Mes)] |
| 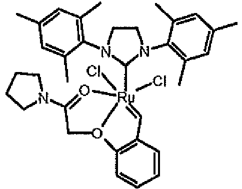 | [RuCl₂(=CH(o-OCH₂CO-*N*-Pyrrolidin)Ph)(ImH₂Mes)] |

21. Verfahren nach den Ansprüchen 18 bis 20, wobei die Ringschlussmetathesereaktion in Schritt a) in einem organischen Lösungsmittel bei 20°C bis 140°C durchgeführt wird.

22. Verfahren nach den Ansprüchen 18 bis 21, wobei die Ringschlussmetathesereaktion in Schritt a) mit einem Verhältnis von Substrat zu Katalysator im Bereich von 20 bis 10000 durchgeführt wird.

23. Verfahren nach den Ansprüchen 19 bis 22, wobei die Hydrolyse in Schritt b) mit einer wässrigen Alkalihydroxidlösung bei einer Temperatur von 0°C bis 40°C durchgeführt wird.

24. Verfahren nach den Ansprüchen 19 bis 23, wobei die makrocyclische Säure der Formel VI, erhalten in Schritt b), durch Extraktion mit Dichlormethan und eine anschließende Kristallisation in Tetrahydrofuran isoliert wird.

25. Verfahren nach den Ansprüchen 19 bis 24, wobei die Bildung des makrocyclischen Sulfonamids der Formel VII in Schritt c) in einem ersten Schritt die Umsetzung der makrocyclischen Säure der Formel VI mit Essigsäureanhydrid in Gegenwart einer anorganischen Base und eines geeigneten organischen Lösungsmittels zu einem Azlacton-Zwischenprodukt der Formel 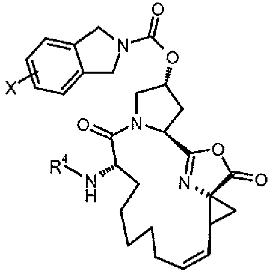 wobei R⁴ eine Amino-Schutzgruppe ist und X Halogen ist, und die anschließende Umsetzung des Azlactons mit Cyclopropylsulfonamid in Gegenwart einer anorganischen Base zu dem makrocyclischen Sulfonamid der Formel VII darstellt.

26. Verfahren nach den Ansprüchen 19 bis 25, wobei die Natriumbase, die zur Behandlung des makrocyclischen Sulfonamids der Formel VII in Schritt d) verwendet wird, Natriumhydroxid, Natriummethylat oder Natriumethoxid ist.

27. Verfahren nach den Ansprüchen 18 bis 26, wobei
R⁴ Boc ist;
R⁵ Ethyl ist;
und die Einheit der Formel 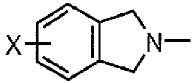 für 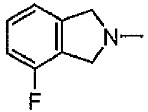 steht,

## Revendications

1. Composé de formule 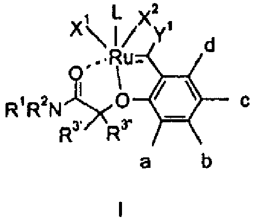 où la ligne pointillée signifie soit l'existence d'une liaison, soit l'absence de liaison;
L est un ligand neutre choisi parmi 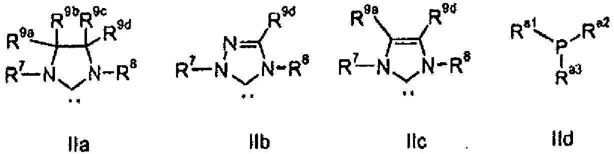 où R⁷ et R⁸, indépendamment l'un de l'autre, sont alkyle en C₁-C₆, aryle, alcényle en C₂-C₆ ou 1-adamantyle, et
R^{9a-d} sont, indépendamment les uns des autres, hydrogène, alkyle en C₁-C₆, alcényle en C₂-C₆ ou aryle, ou R^{9b} et R^{9c} ou R^{9a} et R^{9d}, pris ensemble, forment un pont -(CH₂)₄-;
ou, dans la formule IIc, R^{9a} et R^{9c} sont tous les deux un halogène;
R^{a1}, R^{a2} et R^{a3}, indépendamment les uns des autres, sont alkyle en C₁-C₆, cycloalkyle en C₃-C₈, aryle, hétéroaryle, ou R^{a1} et R^{a2} ou R^{a2} et R^{a3} ou R^{a1} et R^{a3} forment ensemble un groupe cyclooctyle ponté en 1,5;
X¹ et X², indépendamment l'un de l'autre, sont des ligands anioniques;
Y¹ est hydrogène, alkyle en C₁-C₆, cycloalkyle en C₃-C₈, alcényle en C₂-C₆, alcynyle en C₂-C₆, alcoxy en C₁-C₆, alcényloxy en C₂-C₆, alcynyloxy en C₂-C₆, aryloxy, (alcoxy en C₁-C₆)-carbonyle, alkylthio en C₁-C₆, aryle, arylthio, alkylsulfonyle en C₁-C₆, alkylsulfinyle en C₁-C₆;
a, b, c et d, indépendamment les uns des autres, signifient hydrogène, alkyle en C₁-C₆, halogénoalkyle en C₁-C₆, alcényle en C₂-C₆,alcynyle en C₂-C₆, alcoxy en C₁-C₆, alcényloxy en C₂-C₆, alcynyloxy en C₂-C₆, (alkyl en C₁-C₆)-carbonyle, aryle, hydroxy, aryloxy, nitro, (alcoxy en C₁-C₆)-carbonyle, amino, mono(alkyl en C₁-C₆)- ou di(alkyl en C₁-C₆)-amino, halogène, thio, alkylthio en C₁-C₆, alkylsulfonyle en C₁-C₆, alkylsulfinyle en C₁-C₆, arylsulfonyle, SO₃H, (alkyl en C₁-C₆)-carbonylamino, arylcarbonylamino, alkylsulfonylamino en C₁-C₆, arylsulfonylamino, halogénoalkylsulfonylamino en C₁-C₆, SO₃-alkyle en C₁-C₆ ou OSi(alkyle en C₁-C₆)₃ et SO₂-NR'R" où R' et R", indépendamment l'un de l'autre,signifient hydrogène, aryle ou alkyle en C₁-C₆ ou R' et R", ensemble avec l'atome N, forment un cycle;
R¹ et R², indépendamment l'un de l'autre, sont hydrogène, alkyle en C₁-C₆, cycloalkyle en C₃-C₈, aryle, aryl-(alkyle en C₁-C₆) ou
R¹ et R² forment ensemble, avec l'atome N, un cycle de 5 à 8 chaînons qui peut contenir de l'azote, de l'oxygène ou du soufre comme hétéroatome supplémentaire;
R³' et R³", indépendamment l'un de l'autre, sont hydrogène, alkyle en C₁-C₆, cycloalkyle en C₃-C₈, aryle, aryl-(alkyle en C₁-C₆).

2. Composés selon la revendication 1, dans lequel la ligne pointillée représente une liaison et génère un complexe de Ru(II) hexacoordiné.

3. Composé selon la revendication 2, dans lequel L est 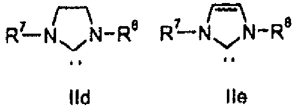 où R⁷ et R⁸, indépendamment l'un de l'autre, sont alkyle en C₁-C₆, 1-adamantyle, un groupe phényle qui est di- ou tri-substitué par alkyle en C₁-C₆, ou naphtyle qui est di- ou tri-substitué par alkyle en C₁-C₆.

4. Composé selon la revendication 3, dans lequel R⁷ et R⁸ sont 2,4,6-triméthylphényle ou 2,7-diisopropylnaphtyle.

5. Composé selon la revendication 1 ou 2, dans lequel X¹ et X², indépendamment l'un de l'autre, sont des halogènes.

6. Composé selon la revendication 5, dans lequel X¹ et X² sont chloro.

7. Composé selon la revendication 1 ou 2, dans lequel Y¹ est un hydrogène.

8. Composé selon la revendication 1 ou 2, dans lequel a, b et d sont l'hydrogène.

9. Composé selon la revendication 1 ou 2, dans lequel c est hydrogène, halogène, nitro, (alkyl en C₁-C₆)-carbonylamino, arylcarbonylamino, arylsulfonylamino, alkylsulfonylamino, halogénoalkylsulfonylamino en C₁-C₆, SO₂-NR'R" où R' et R" signifient, indépendamment l'un de l'autre, hydrogène, alkyle en C₁-C₆, aryle, ou R' et R" ensemble, avec l'atome N, forment un cycle,

10. Composé selon la revendication 1 ou 2, dans lequel R¹ et R², indépendamment l'un de l'autre, sont hydrogène, alkyle en C₁-C₆, ou
R¹ et R², avec l'atome N, forment ensemble un cycle de 6 chaînons qui contient de l'oxygène comme hétéroatome supplémentaire.

11. Composé selon les revendications 1 ou 2, dans lequel R^{3'} et R^{3"}, indépendamment l'un de l'autre, sont hydrogène ou alkyle en C₁-C₆.

12. Composés selon les revendications 1 à 11, choisis parmi
| | |
|---|---|
| 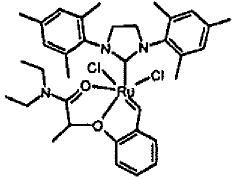 | [RuCl₂(=CH(o-OCH(Me)CONEt₂)Ph)(ImH₂Mes)] |
| 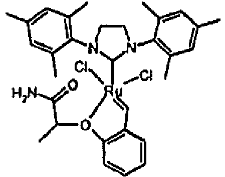 | [RuCl₂(=CH(o-OCH(Me)CONH₂)Ph)(ImH₂Mes)] |
| 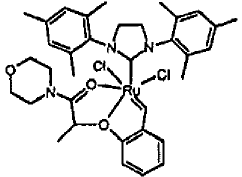 | [RuCl₂(=CH(OOOCH(Me)CO-*N*-Morpholine)Ph)(ImH₂Mes)] |
| 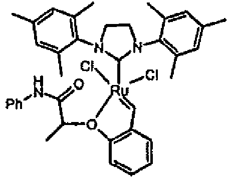 | [RuCl₂(=CH(o-OCH(Me)CONHPh)Ph)(ImH₂Mes)] |
| 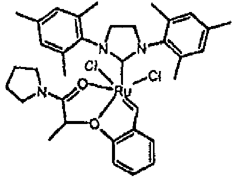 | [RuCl₂(=CH(o-OCH(Me)CO-N-Pyrrolidine)Ph)(ImH₂Mes)] |
| 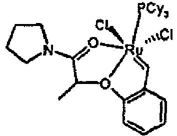 | [RuCl₂(-CH(o-OCH(Me)CO-*N*-Pyrrolidine)Ph)(PCy₃)] |
| 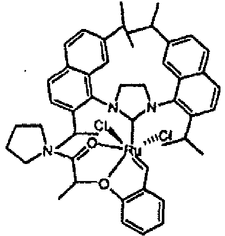 | [RuCl₂(-CH(o-OCH(Me)CO-N-Pyrrolidine)Ph)(SIPrNap)] |
| 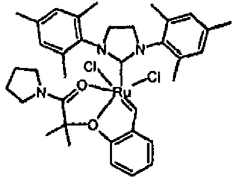 | [RuCl₂(=CH(o-OCMe₂CO-N-Pyrrolidine)Ph)(ImH₂Mes)] |
| 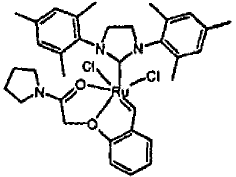 | (RuCl₂(=CH(o=OCH₂CO-N-Pyrrolidine)Ph)(ImH₂Mes)] |

13. Procédé de préparation de composés de formule I, comprenant la conversion d'un préligand de formule 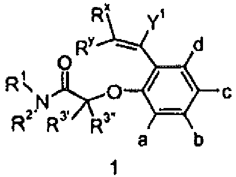 où R¹, R² et R^{3'} et R^{3"}, Y¹ et a, b, c, d sont tels que définis ci-dessus;
R^{x} et R^{y}, indépendamment l'un de l'autre, désignent l'hydrogène, un alkyle en C₁-C₆ éventuellement substitué par un ou plusieurs atomes d'halogène ou un aryle éventuellement substitué par un ou plusieurs atomes d'halogène ou par alkyle en C₁-C₆,
avec un complexe de Ru choisi parmi 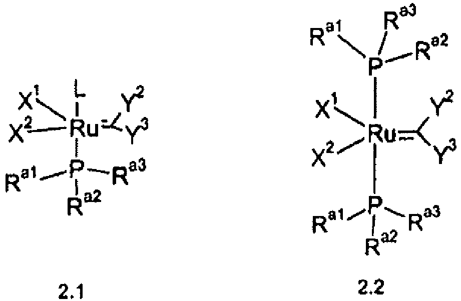 où L, X¹ et X² sont tels que définis ci-dessus;
Y² et Y³, indépendamment l'un de l'autre, sont hydrogène, alkyle en C₁-C₆, alcényle en C₂-C₆, alcynyle en C₂-C₆, alkylthio en C₁-C₆, aryle, arylthio, alkylsulfonyle en C₁-C₆, alkylsulfinyle en C₁-C₆,
ou Y² et Y³ pris ensemble forment un cycle du type 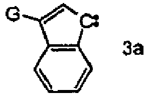 G étant hydrogène ou aryle;
ou
Y² et Y³ forment ensemble un groupe cumulényle du type 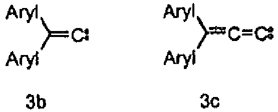 R^{a1}, R^{a2} et R^{a3}, indépendamment les uns des autres, sont alkyle en C₁-C₆, cycloalkyle en C₃-C₈, aryle, hétéroaryle, ou R^{a1} et R^{a2} ou R^{a2} et R^{a3} ou R^{a1} et R^{a3} forment ensemble un groupe cyclooctyle ponté en 1,5.

14. Procédé selon la revendication 13, **caractérisé en ce que** la conversion s'effectue dans un solvant inerte à une température de 0°C à 80°C.

15. Procédé selon les revendications 13 et 14, **caractérisé en ce que** la conversion s'effectue en présence de CuCl.

16. Préligands de formule 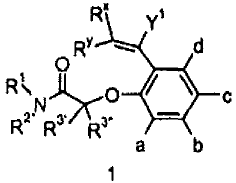 où R¹, R² et R^{3'} et R^{3"}, R^{x}, R^{y}, Y¹ et a, b, c, d sont tels que définis ci-dessus, choisis parmi

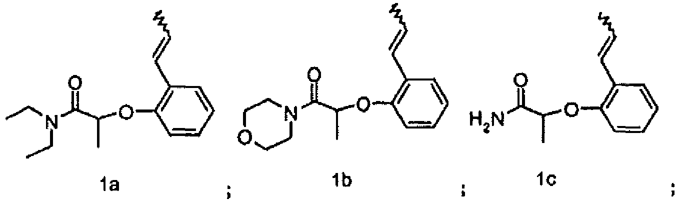

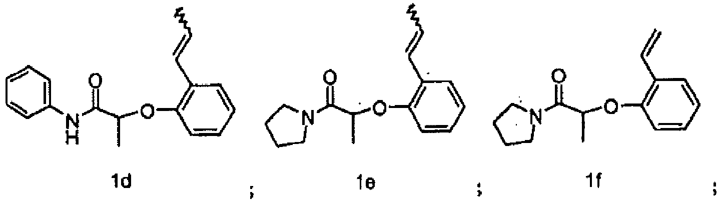

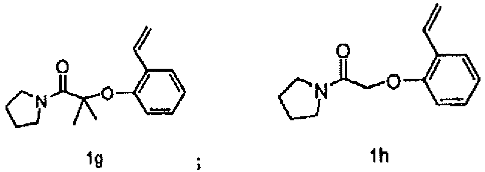

17. Utilisation des composés des revendications 1 à 12 dans des réactions de métathèse.

18. Procédé de préparation d'un composé macrocyclique de formule 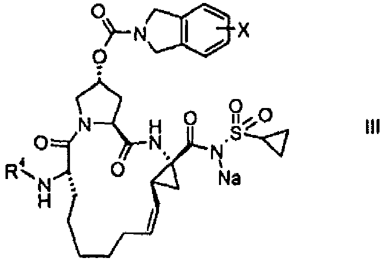 où R⁴ est un groupe protecteur d'amino et X est un atome d'halogène, dans lequel on soumet un composé diène de formule 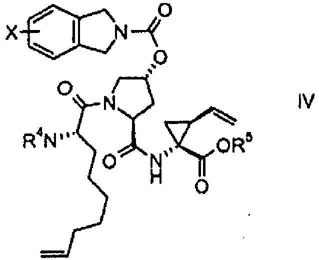 où R⁴ est un groupe protecteur d'amino, R⁵ est un alkyle en C₁-C₄ et X est un halogène,
à une réaction de cyclisation par métathèse en présence d'un composé des revendications 1 à 12.

19. Procédé de préparation d'un composé macrocyclique de formule 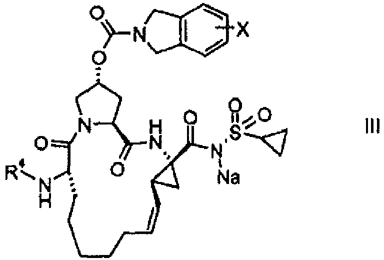 où R⁴ est un groupe protecteur d'amino et X est un atome d'halogène, comprenant les étapes selon lesquelles
a) on soumet un composé diène de formule 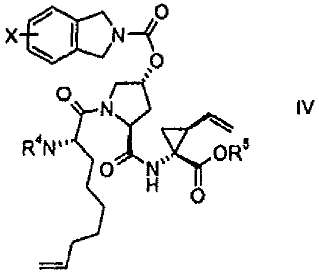 où R⁴ est un groupe protecteur d'amino, R⁵ est un alkyle en C₁-C₄ et X est un halogène, à une réaction de cyclisation par métathèse en présence d'un composé des revendications 1 à 12
pour former un ester macrocyclique de formule 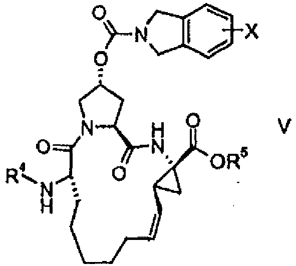 où R⁴ est un groupe protecteur d'amino, R⁵ est un alkyle en C₁-C₄ et X est un halogène;
b) on hydrolyse l'ester macrocyclique de formule V en présence d'une base pour former l'acide macrocyclique de formule 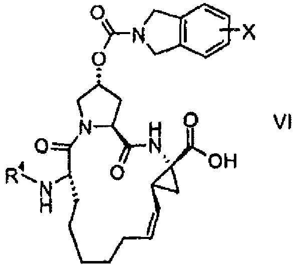 où R⁴ est un groupe protecteur d'amino et X est un halogène;
c) on forme le sulfonamide macrocyclique de formule 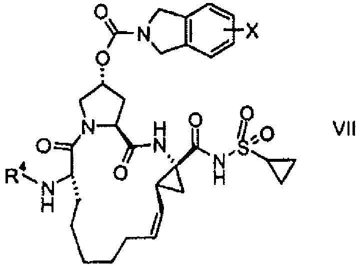 où R⁴ est un groupe protecteur d'amino et X est un halogène, par couplage de l'acide macrocyclique de formule VI avec du cyclopropylsulfonamide, et
d) on traite le sulfonamide macrocyclique de formule XXI avec une base de sodium pour former le composé macrocyclique de formule III.

20. Procédé selon la revendication 18 ou 19, où les composés des revendications 1 à 12 sont choisis parmi les composés:
| | |
|---|---|
| 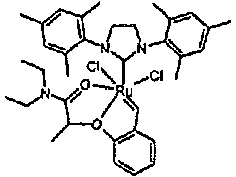 | [RuCI₂(-CH(o-OCH(Me)CONEt₂)Ph)(ImH₂Mes)] |
| 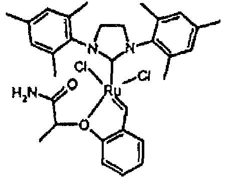 | [RuCl₂(-CH(o-OCH(Me)CONH₂Ph)(ImH₂Mes)] |
| 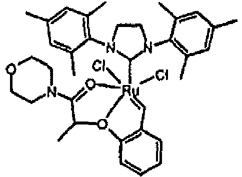 | [RuCl₂(=CH(o-OCH(Me)CO-N-Morpholine)Ph)(ImH₂Mes)] |
| 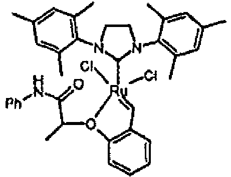 | [RuCl₂(=CH(o-OCH(Me)CONHPh)Ph)(ImH₂Mes)] |
| 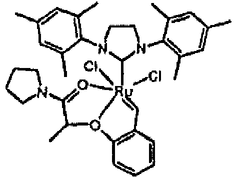 | [RuCl₂(=CH(o-OCH(Me)CO-*N*-Pyrolidine)Ph)(ImH₂Mes)] |
| 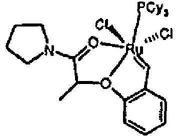 | [RuCl₂(=CN(o-OCH(Me)CO-*N*-Pyrrolidine)Ph)(PCy₃)] |
| 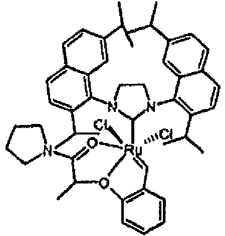 | [RuCl₂(=CH(o.OCH(Me)CO-*N*-Pyrrolidine)Ph)(SIPrNap)] |
| 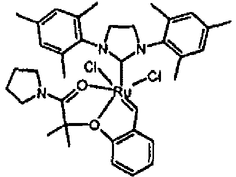 | [RuCl₂(=CH(o-OCMe₂CO-*N*-Pyrrolidine)Ph)(ImH₂Mes)] |
| 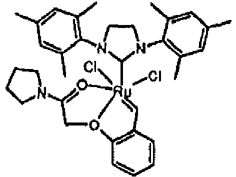 | [RuCl₂(=CH(o-OCH₂CO-*N*-Pyrrolidine)Ph)(ImH₂Mes)] |

21. Procédé selon les revendications 18 à 20, dans lequel la réaction de cyclisation par métathèse dans l'étape a) s'effectue dans un solvant organique à 20°C à 140°C.

22. Procédé selon les revendications 18 à 21, dans lequel la réaction de cyclisation par métathèse dans l'étape a) s'effectue avec un rapport du substrat au catalyseur se situant dans le domaine de 20 à 10 000.

23. Procédé selon les revendications 19 à 22, dans lequel l'hydrolyse dans l'étape b) s'effectue avec une solution aqueuse d'hydroxyde de métal alcalin à une température de 0°C à 40°C.

24. Procédé selon les revendications 19 à 23, dans lequel on isole l'acide macrocyclique de formule VI obtenu dans l'étape b) au moyen d'une extraction avec du dichlorométhane, suivie d'une cristallisation dans du tétrahydrofurane.

25. Procédé selon les revendications 19 à 24, dans lequel la formation du sulfonamide macrocyclique de formule VII dans l'étape c) est, dans une première étape, la réaction de l'acide macrocyclique de formule VI avec de l'anhydride acétique en présence d'une base inorganique et d'un solvant organique approprié, donnant une azlactone intermédiaire de formule 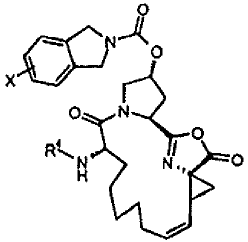 où R⁴ est un groupe protecteur d'amino et X est un halogène, puis la réaction de l'azlactone avec du cyclopropylsulfonamide en présence d'une base inorganique, donnant le sulfonamide macrocyclique de formule VII.

26. Procédé selon les revendications 19 à 25, dans lequel la base de sodium utilisée pour le traitement du sulfonamide macrocyclique de formule VII dans l'étape d) est l'hydroxyde de sodium, le méthylate de sodium ou l'éthylate de sodium.

27. Procédé selon les revendications 18 à 26, dans lequel
R⁴ est Boc;
R⁵ est éthyle;
et le groupe de formule 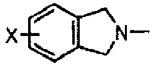 représente le groupe
